# EUROPEAN PATENT APPLICATION

(11) **EP 2 559 373 A1**
(43) Date of publication of application: **20.02.2013**
(21) Application number: 11768939.8
(22) Date of filing: 14.04.2011
(51) Int. Cl.: A61B 5/00, A61B 5/145, G01N 27/416, G06Q 50/00

(54) **BLOOD SUGAR LEVEL MEASUREMENT DEVICE, BLOOD SUGAR LEVEL MEASUREMENT RESULT DISPLAY METHOD, AND BLOOD SUGAR LEVEL MEASUREMENT RESULT DISPLAY CONTROL PROGRAM**

(30) Priority: 14.04.2010 JP 2010093575
(71) Applicant: Arkray, Inc., Minami-ku Kyoto-shi Kyoto 601-8045 (JP)
(72) Inventor: NISHIYAMA, Hisashi, Kyoto-shi Kyoto 602-0008 (JP); SAKATA, Tetsuya, Kyoto-shi Kyoto 602-0008 (JP); SHIMIZU, Takeshi, Kyoto-shi Kyoto 602-0008 (JP)
(74) Representative: MacDougall, Alan John Shaw
(86) International application number: PCT/JP2011/059316
(87) International publication number: WO 2011/129418

(57) **Abstract**

A measured result reflecting a body condition of a user when measuring a blood sugar level can be displayed, and convenience of management of the blood sugar level of the user is improved. In a blood sugar level measuring apparatus including: a measuring unit to measure a blood sugar level of a user; a recording unit to record measurement data thereof; and a display unit to capable of displaying a measured result on a display, the improvement characterized by further comprising: a condition flag setting unit to record, on the recording unit, a condition flag related to a body condition of the user when measuring the blood sugar level in a way that associates the condition flag with blood sugar level data measured by the measuring unit; and a display control unit to display the measured result recorded on the recording unit on the display on the basis of the condition flag set by the condition flag setting unit.

## Description

### TECHNICAL FIELD

The present invention relates to a blood sugar level measuring apparatus which measures a blood sugar level of a user, a blood sugar level measured result display method thereof and a blood sugar level measured result display control program.

### BACKGROUND ART

Operability of a measuring apparatus and preparations etc required for the measurement are extremely large problems to a user such as a diabetic who has to measure the blood sugar level and control an amount of meal and an amount of exercise in terms of health management. Under such circumstances, a portable measuring apparatus enabling the user to measure the blood sugar level comparatively easily in a daily life is developed and mounted with a variety of functions that assist the use in the health management. For example, a technology disclosed in Patent document 1 is configured to enable the user to easily recognize transitions of the blood sugar levels by displaying graphs of recent measurement values together with the past measurement values on a display of the measuring apparatus.

### PRIOR ART DOCUMENT

### PATENT DOCUMENT

Patent document 1: Japanese National Publication of International Patent Application No. 2005-106446

### SUMMARY OF THE INVENTION

### PROBLEMS TO BE SOLVED BY THE INVENTION

The user having a necessity of managing the blood sugar level in terms of the health is requested to measure the blood sugar level of the user himself or herself at a proper point of time under a variety of situations of the normal life. On the other hand, it is recognized that the blood sugar level has a tendency of increasing and decreasing in value corresponding to a body condition of the user, e.g., conditions such as a pre-meal condition, a post-meal condition and a post-exercise condition, and it can be therefore a highly important technical element in terms of managing the blood sugar level of the user to recognize how the body condition is when the blood sugar level for the measurement is measured.

In the conventional technologies, however, though a measurement history of the past blood sugar levels is displayed by graphs, the body condition of the user when making the measurement is not reflected in the graph. It is therefore not said that convenience of the management of the blood sugar level of the user is sufficiently improved.

It is an object of the present invention, which was devised in view of the problems described above, to provide a blood sugar level measuring apparatus, a blood sugar level measured result display method thereof and a blood sugar level measured result display control program that are capable of displaying a measured result reflecting a body condition of a user when measuring a blood sugar level and improving convenience of managing the blood sugar level of the user.

### MEANS FOR SOLVING THE PROBLEMS

The present invention takes, in order to solve the problems described above, a configuration of managing measured blood sugar level data and a condition flag representing a body condition of a user when making the measurement in a way that associates the blood sugar level data and the condition flag with each other, and displaying the measured result on a display on the basis of this associative relation, thereby enabling the improvement of the convenience with which user manages the blood sugar level.

Specifically, according to the present invention, in a blood sugar level measuring apparatus including: a measuring unit to measure a blood sugar level of a user; a recording unit to record measurement data thereof; and a display unit to capable of displaying a measured result on a display, the improvement is characterized by further including: a condition flag setting unit to record, on the recording unit, a condition flag related to a body condition of the user when measuring the blood sugar level in a way that associates the condition flag with blood sugar level data measured by the measuring unit; and a display control unit to display the measured result recorded on the recording unit on the display on the basis of the condition flag set by the condition flag setting unit.

The blood sugar level measuring apparatus according to the present invention measures the blood sugar level of the user and displays the measured result thereof, thereby improving the convenience with which user manages the blood sugar level. The condition flag setting unit associates the condition flag related to the body condition of the user when making the measurement with the blood sugar level data measured by the measuring unit, and thereafter the measurement data is managed within the apparatus. It therefore follows that the display control unit controls the display of the measured result in a status where the condition flag is associated with the measurement data.

According to the configuration such as this, the data management is conducted by associating the condition flag defined as information on the body condition that largely affects the blood sugar level of the user with the measurement data within the blood sugar level measuring apparatus, and hence the user can confirm how the body condition is when measuring the blood sugar level for measurement through a display content of the display control unit, whereby determination based on the measured result is properly made, and the convenience of the management of the blood sugar level can be improved. If based on a point that the blood sugar level in a blood largely fluctuates depending on the body condition, it is greatly beneficial to the user that the data are managed by thus associating the condition flag with the measurement data.

In the present invention, the condition flag is formed so that a predetermined portion can be, e.g., lit up to indicate a predetermined shape on the display; the condition flag setting unit may display the predetermined portion on the display by lighting up this predetermined portion when a predetermined case arises; further, e.g., the blood sugar level measuring apparatus may include a memory, and predetermined flags may be previously stored in this memory, or alternatively desired flags may also be stored therein according to the request; and the condition flag setting unit may properly invoke the stored flag and may display the flag in the way of being associated with the corresponding measurement data. Namely, in the present invention, the condition flags may be fixedly set beforehand in the blood sugar level measuring apparatus and may also be changed such as being added or deleted according to the request. The blood sugar level measuring apparatus according to the present invention may include any one of the configuration that the flags are thus fixedly set and the configuration that these flags can be changed according to the request, and may also include both of these configurations. Moreover, the blood sugar level measuring apparatus according to the present invention includes a memory capable of adding or deleting the flags; the condition flag setting unit records biometric information of the user in relation to the blood sugar level of the user on the recording unit; the blood sugar level measuring apparatus further includes a matching unit to accept input of the biometric information and match the information managed by the blood sugar level measuring apparatus with at least a part of information managed by an external apparatus; and the display control unit displays the biometric information recorded on the recording unit on the display. According to these configurations, the information managed by the blood sugar level measuring apparatus can be matched with the information managed by the external apparatus. For example, if the external apparatus is installed at a hospital where the user undergoes a diagnosis, management items on the side of the hospital can be matched with the management items on the user's side, and, in the items managed at the hospital, the items desired to be managed on the user's side can be contained as the management items of the blood sugar level measuring apparatus, which is highly effective in the diagnosis and a medical treatment.

Herein, by way of one example of the display control by the display control unit, the display control unit may be configured to display graphs of transitions of the blood sugar levels of the user on the display on the basis of the measurement data recorded on the recording unit. Further, the display control unit may display predetermined marks corresponding to the respective condition flags associated with plural pieces of blood sugar level data indicated by the graphs in the way of being adjacent to the graphs. The user can easily understand fluctuations of his or her blood sugar levels and gets easy to be aware of caution points about the health by displaying the graphs of the transitions of the blood sugar levels. Further, predetermined marks corresponding to the condition flags set by the condition flag setting unit are displayed adjacent to the displayed graphs, whereby the user can recognize the transitions of the blood sugar levels and the body conditions simultaneously. This contrivance is highly useful in terms of the convenience to the user. Note that the predetermined marks are different corresponding to types of the condition flags to be set, however, if based on the point of being displayed together with the graphs on the display, it is preferable to determine shapes, colors, etc thereof so as to be distinguishable between the marks when the user glances at the marks.

As another example of the display control by the display control unit, the display control unit may display an arithmetic value based on the measurement data recorded on the recording unit on the display. In this case, further, the display control unit may extract a measurement data group exhibiting the same condition flag set by the condition flag setting unit from within the measurement data recorded on the recording unit, and may display the arithmetic value about the extracted measurement data group on the display.

The arithmetic value is an arithmetic value calculated based on the measured result and becoming a useful piece of information in terms of managing the blood sugar level of the user. For instance, the arithmetic value is exemplified by an average value of the blood sugar levels of the user for a predetermined period but is not limited to only this average value. On the other hand, if based on such a point that the blood sugar level has the tendency of fluctuating up and down depending on the body condition of the user, as described above, it is considered helpful in managing the blood sugar level of the user to display the arithmetic value per condition flag related to the body condition. For instance, the arithmetic value of the post-meal blood sugar level and the arithmetic value of the pre-meal blood sugar level can be displayed in distinction therebetween rather than displaying the arithmetic values with the post-meal and pre-meal blood sugar levels being mixed, and hence the user can make the determination about the blood sugar level more accurately and easier. In the present invention, as described above, the condition flag is managed in the way of being associated with the measurement data, whereby the variety of displays such as displaying the graphs and displaying the arithmetic value can be easily controlled.

Note that the condition flags associated by the condition flag setting unit in the blood sugar level measuring apparatus may be whatever flags representing phenomena and events related to conditions of a living body. These flags contain flags etc representing facts pertaining to actions on the living body in addition to the flags representing the conditions of the living body itself such as the flags indicating a pre-meal condition, a post-meal condition and a post-exercise condition of the user, and whatever condition flags can be adopted if being the flags related to the body conditions related to the blood sugar levels of the user. These condition flags contain, e.g., flags indicating the facts related to the actions taken by the user such as the pre-meal condition, the post-meal condition, the amount of meal, the post-exercise condition and the amount of exercise of the user, flags indicating physiological/biochemical conditions of the living body such as flags representing an in-vivo substance quantity of the user like an amount of β cells, a cholesterol value and a Hb (haemoglobin) A1c value and changes thereof, flags indicating whether or not a medicine is dosed such as dosing insulin to the user and how much degree of medicine is dosed, and flags indicating the facts related to the actions on the living body. Further, the "condition" is generally interpreted as "a state at a certain point of time" in some cases, however, the present description gives a connotation "state" in a broad sense, and the "condition" embraces not only a change at a certain point of time but also a change with time elapse in the living body and a degree of the change as well. Furthermore, the "condition" contains not only the action on the living body and whether the action on the living body is taken or not but also a degree of the action. Further, the condition flag may contain whatever biometric information managed in relation to the blood sugar level at a management institution such as the hospital. The information managed at the hospital etc can be matched with the information grasped by the user, and it is feasible to improve accuracy of associating the measured blood sugar level with the biometric information managed in relation to the blood sugar level.

Herein, if the condition flag embraces a concept of "degree", the condition flag may be formed to indicate an increase and a decrease of this degree by incrementing and decrementing the number of predetermined marks. For instance, if the condition flag is the flag indicating the amount of meal, the increase and decrease in amount of meal may be displayed by incrementing and decrementing, e.g., the number of bar marks. That is, the blood sugar level measuring apparatus may adopt a configuration that if the condition flag associated by the condition flag setting unit is the flag about the post-meal condition, the display control unit displays information on an amount of meal of the user together with the blood sugar level of a blood of the user, which is measured by the measuring unit, on the display. This configuration takes account of such a point that even when the condition flag representing the post-meal condition of the user is set, influence exerted on the blood sugar level of the "meal" largely fluctuates depending on the amount thereof. For example, the information on the amount of meal of the user may be displayed as an image imitating the stomach of a person and as a variation image that varies corresponding to the amount of meal, and, e.g., the variation image may vary as the number of bar marks changes.

Furthermore, the blood sugar level measuring apparatus may, if the condition flag associated by the condition flag setting unit is the flag representing the condition of the living body that changes with the elapse time, adopt the following configuration. For example, the display control unit may display, on the display, information on the elapse time since at least one of a point of time when taking action on the living body and starting the action on the living body, a point of time when finishing the action on the living body and a predetermined point of time during a period from the starting point of time to the finishing point of time together with the blood sugar level of the blood of the user, which is measured by the measuring unit. Further, by way of another example, the blood sugar level measuring apparatus may adopt a configuration that if the condition flag associated by the condition flag setting unit is the flag about the post-meal flag, the display control unit may display, on the display, information on elapse time since at least one of a point of time when the user starts having the meal, a predetermined point of time during the meal and a point of time when completing the meal together with the blood sugar level of the blood of the user that is measured by the measuring unit. Similarly to the amount of meal, the elapse time since these predetermined points of time is also a parameter exerting the influence on the increase and the decrease in blood sugar level of the user. Such being the case, in the present invention, the elapse time is thus reflected in the blood sugar level data, whereby the convenience with which user manages the blood sugar level can be further improved.

Herein, the blood sugar level measuring apparatus may be configured to further include an operation unit through which the user can give an instruction to associate the condition flag with the blood sugar level data, and, in this case, the condition flag setting unit further may associate the information on the amount of meal with the blood sugar level data may be associated with the flag about the post-meal condition on the basis of a user's operation over the operation unit. That is, the information on the amount of meal is handled through the user' s operation on the operation unit.

Here, the operation unit may have a function of an operation of inputting or selecting a predetermined condition about contents of the meal, and, in such a case, the condition flag setting unit may automatically calculate the information on the amount of meal according to the result of the operation, and associates this information with the blood sugar level data. According to this configuration, the user performs the operation about the predetermined condition related to the contents of the meal that are easy to input and select comparatively objectively, thereby automatically calculating the information on the amount of meal. Therefore, the operability of the blood sugar level measuring apparatus can be improved, and the accuracy of the information on the amount of meal can be also increased.

Next, the present invention can be grasped from an aspect of a blood sugar level measured result display method. Namely, the present invention is a blood sugar level measured result display method of displaying, in a blood sugar level measuring apparatus including: a measuring unit to measure a blood sugar level of a user; a recording unit to record measurement data thereof; and a display unit to capable of displaying a measured result on a display, the measured result on the display, the method including: a condition flag setting step of recording, on the recording unit, a condition flag related to a body condition of the user when measuring the blood sugar level in a way that associates the condition flag with measured blood sugar level data; and a display control step of displaying the measured result recorded on the recording unit on the display on the basis of the condition flag set in the condition flag setting step. Also in the display method according to the present invention, as in the case of the blood sugar level measuring apparatus, the body condition of the user is reflected in the measured result, thereby enabling the improvement of the convenience with which user manages the blood sugar level.

Moreover, in the blood sugar level measured result display method, the display control step may include displaying graphs of transitions of the blood sugar levels of the user on the display on the basis of the measurement data recorded on the recording unit. Moreover, the display control step may include displaying predetermined marks corresponding to the respective condition flags associated with plural pieces of blood sugar level data indicated by the graphs in the way of being adjacent to the graphs.

Further, the display control step may include displaying an arithmetic value based on the measurement data recorded on the recording device on the display. Still further, the display control step may include extracting a measurement data group exhibiting the same condition flag set by the condition flag setting unit from within the measurement data recorded on the recording unit, and displaying the arithmetic value about the extracted measurement data group on the display. Note that an average value of the blood sugar levels of the user for a predetermined period can be given by way of one example of this arithmetic value.

Also in the thus-configured blood sugar level measured result display method, as in the case of the blood sugar level measuring apparatus, the body condition of the user is reflected in the measured result and can be thus displayed on the display, whereby the convenience with which user manages the blood sugar level can be improved. It is to be noted that the technical idea disclosed with respect to the blood sugar level measuring apparatus can be similarly applied to the display method according to the present invention.

Next, the present invention can be also grasped from an aspect of a blood sugar level measured result display control program. Namely, the present invention is a blood sugar level measured result display control program as a computer program to make a computer including: a measuring unit to measure a blood sugar level of a user; a recording unit to record measurement data thereof; and a display unit to capable of displaying a measured result on a display, display the measured result on the display, the program making the computer execute: a condition flag setting step of recording, on the recording unit, a condition flag related to a body condition of the user when measuring the blood sugar level in a way that associates the condition flag with measured blood sugar level data; and a display control step of displaying the measured result recorded on the recording unit on the display on the basis of the condition flag set in the condition flag setting step. Also in the display control program, as in the case of the blood sugar level measuring apparatus, the body condition of the user is reflected in the measured result and can be thus displayed on the display, whereby the convenience with which user manages the blood sugar level can be improved.

Moreover, in the blood sugar level measured result display control program, the display control step may include displaying graphs of transitions of the blood sugar levels of the user on the display on the basis of the measurement data recorded on the recording unit. Furthermore, the display control step may include displaying predetermined marks corresponding to the respective condition flags associated with plural pieces of blood sugar level data indicated by the graphs in the way of being adjacent to the graphs.

Further, the display control step may include displaying an arithmetic value based on the measurement data recorded on the recording unit on the display. Still further, the display control step may include extracting a measurement data group exhibiting the same condition flag set by the condition flag setting unit from within the measurement data recorded on the recording unit, and displaying the arithmetic value about the extracted measurement data group on the display. Note that the average value of the blood sugar levels of the user for the predetermined period can be given by way of one example of this arithmetic value.

Also in the thus-configured blood sugar level measured result display control program, as in the case of the blood sugar level measuring apparatus, the body condition of the user is reflected in the measured result and can be thus displayed on the display, whereby the convenience with which user manages the blood sugar level can be improved. It is to be noted that the technical idea disclosed with respect to the blood sugar level measuring apparatus can be similarly applied to the display control program according to the present invention.

Moreover, a computer readable recording medium recorded with the display control program is also embraced by the category of the present invention.

### EFFECTS OF THE INVENTION

The measured result reflecting the body condition of the user when measuring the blood sugar level can be displayed, and the convenience with which user manages the blood sugar level can be improved.

### BRIEF DESCRIPTION OF THE DRAWINGS

[FIG.1A] A first view illustrating an external configuration of a blood sugar level measuring apparatus according to the present invention.
[FIG.1B] A second view illustrating an external configuration of the blood sugar level measuring apparatus according to the present invention.
[FIG.2] A functional block diagram of the respective function units configured in the blood sugar level measuring apparatus illustrated in FIG. 1.
[FIG.3] A flowchart of a blood sugar level measuring process executed in the blood sugar level measuring apparatus illustrated in FIG. 1.
[FIG.4] A view illustrating a display example of a liquid crystal screen of the blood sugar level measuring apparatus illustrated in FIG. 1.
[FIG.5] A flowchart of a measured result management process executed in the blood sugar level measuring apparatus illustrated in FIG. 1.
[FIG. 6] A flowchart of a graph display process executed in the blood sugar level measuring apparatus illustrated in FIG. 1.
[FIG.7A] A first view illustrating graphs displayed on the liquid crystal screen of the blood sugar level measuring apparatus in the graph display process illustrated in FIG 6.
[FIG.7B] A second view illustrating the graphs displayed on the liquid crystal screen of the blood sugar level measuring apparatus in the graph display process illustrated in FIG 6.
[FIG.8] A flowchart of a measurement history display process executed in the blood sugar level measuring apparatus illustrated in FIG. 1.
[FIG.9] A first view of a display example displayed on the liquid crystal screen of the blood sugar level measuring apparatus in the measurement history display process illustrated in FIG 8.
[FIG.10] A second view of the display example displayed on the liquid crystal screen of the blood sugar level measuring apparatus in the measurement history display process illustrated in FIG 8.
[FIG.11] A third view of the display example displayed on the liquid crystal screen of the blood sugar level measuring apparatus in the measurement history display process illustrated in FIG 8.
[FIG.12] A flowchart of the measured result management process executed in the blood sugar level measuring apparatus illustrated in FIG. 1.
[FIG.13] A flowchart of the measured result management process executed in the blood sugar level measuring apparatus illustrated in FIG. 1.
[FIG.14] A flowchart of the measured result management process executed in the blood sugar level measuring apparatus illustrated in FIG. 1.
[FIG.15] A view illustrating the display example of the liquid crystal screen of the blood sugar level measuring apparatus illustrated in FIG. 1.
[FIG.16] A view illustrating the display example of the liquid crystal screen of the blood sugar level measuring apparatus illustrated in FIG. 1.
[FIG.17] A view illustrating the display example of the liquid crystal screen of the blood sugar level measuring apparatus illustrated in FIG. 1.
[FIG.18] A view illustrating the display example of the liquid crystal screen of the blood sugar level measuring apparatus illustrated in FIG. 1.
[FIG.19] A view illustrating the display example of the liquid crystal screen of the blood sugar level measuring apparatus illustrated in FIG. 1.
[FIG.20] A diagram of a system architecture of an information processing system according to the present invention.
[FIG.21] A flowchart of a management information matching process executed in the blood sugar level measuring apparatus illustrated in FIG. 1.

### [Mode for Carrying out the Invention]

A blood sugar level measuring apparatus according to a mode for carrying out the present invention will hereinafter be described with reference to the drawings. Note that a configuration in the following embodiment is an exemplification, and the present invention is not limited to the configuration in the embodiment.

### [First Working example]

### <Outline of Blood Sugar Level Measuring Apparatus>

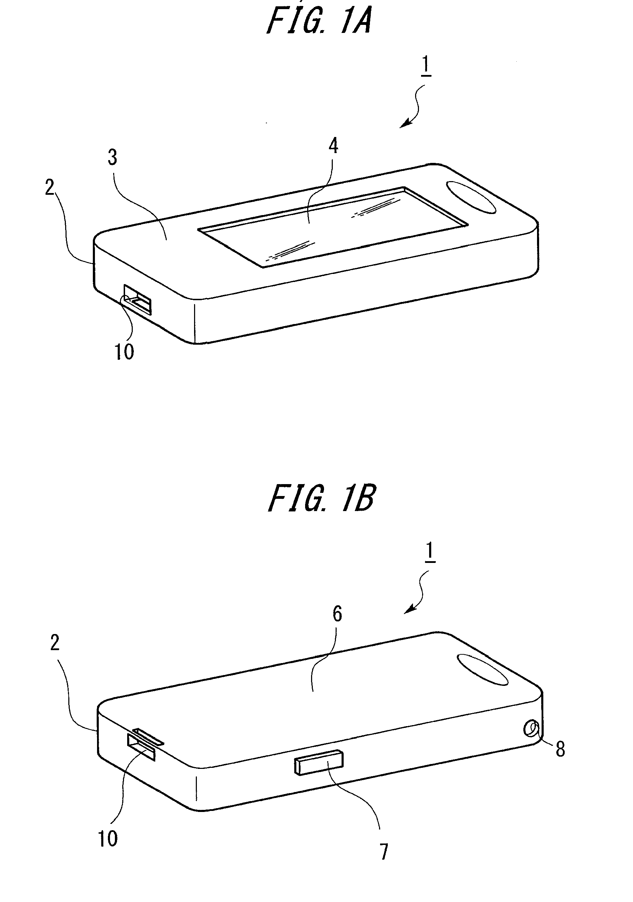

FIG. 1 is a view illustrating an external configuration of a blood sugar level measuring apparatus 1 according to the present invention. The blood sugar level measuring apparatus 1 is a portable measuring apparatus configured to enable a user having diabetes mellitus to measure a blood sugar by himself or herself. As depicted in FIG. 1, the blood sugar level measuring apparatus 1 is configured to include an elongate housing 2 having a size that is as large as a palm of the user, which aims at improving usability of the user and enables the user to manage and display a measured result properly and quickly as will be mentioned later on, thus being instrumental to the management of the blood sugar of the user.

To be specific, FIG. 1A depicts a configuration on the surface side of the blood sugar level measuring apparatus 1, while FIG. 1B illustrates a configuration on the underside thereof. A liquid crystal screen 4 for displaying the measured result etc is provided on a panel 3 on the surface side of the housing 2, however, a display process thereon will be described later on. Further, an edge portion of the housing 2 is formed with a test strip insertion groove 10 into which a measurement test strip (unillustrated) used in the blood sugar level measuring apparatus 1 is inserted. This test strip is provided with a sensor that reacts on glucose in a blood sampled from the user and outputs an electric signal, and it follows that the user adheres a droplet of user's blood to this sensor when measuring a blood sugar level, and the blood sugar level measuring apparatus 1 measures this blood sugar level. The sensor given above can involve adopting a sensor that uses, e.g., glucose oxidase (GOD) as oxidoreductase and a hydrogen peroxide electrode as an electrode for outputting the signal. A configuration of the test strip is based on the prior arts but is not a core element of the present invention, and hence an in-depth description thereof is omitted in the present description.

Further, as illustrated in FIG. 1B, a side face of the panel 3 on the underside of the housing 2 is provided with an operation button 7 for operating the blood sugar level measuring apparatus 1 and a communication port 8 which establishes a connection enabled to perform communications between the blood sugar level measuring apparatus 1 and an external apparatus, e.g., a computer etc disposed outside. The operation button 7 enables the user to conduct operations for all processes executed in the blood sugar level measuring apparatus 1 by combining a variety of operations such as a pressing operation, a slide operation and a pressing state retaining operation.

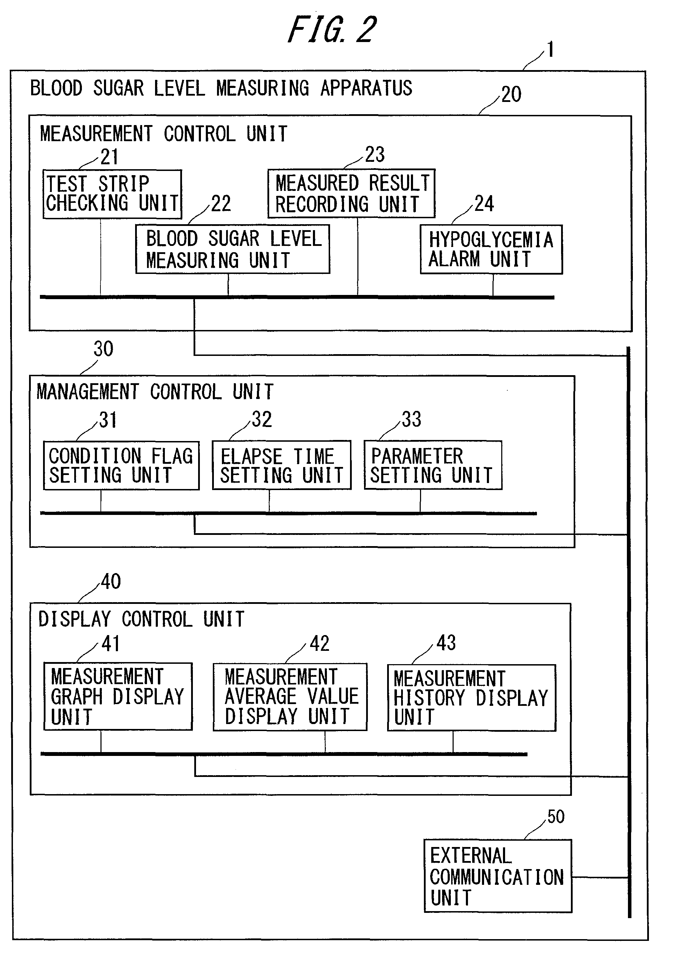

Further, an interior of the blood sugar level measuring apparatus 1 is provided with control units for controlling a variety of processes executed therein. The control units provided as main control units in the blood sugar level measuring apparatus 1 are, as imaged in FIG. 2, a measurement control unit 20, a management control unit 30, a display control unit 40 and an external communication unit 50; and these control units are configured within the blood sugar level measuring apparatus 1 and realized by a computer program executed on a computer including a CPU (Central Processing Unit), a memory, a hard disk, etc, which are not illustrated herein. Furthermore, the liquid crystal screen 4, the operation button 7 and the communication port 8 described above are also properly connected to the respective control units illustrated in FIG. 2, thereby executing processes of the program related to displaying the measured result to the user, transmitting an operating instruction given from the user to the measuring apparatus, communicating the data accumulated within the blood sugar level measuring apparatus 1 to the outside, and so on. Detailed configurations of the control units of the blood sugar level measuring apparatus 1 according to the present invention will hereinafter be described.

As described above, the control units of the blood sugar level measuring apparatus 1 are built up mainly by the measurement control unit 20 which performs measurement control pertaining to the measurement of the blood sugar level, the management control unit 30 which conducts management control concerning items of information on the measured result, a flag, etc, that are managed by the blood sugar level measuring apparatus 1, the display control unit 40 which carries out display control of the measurement result and the external communication unit 50 which performs control of the communications with the outside of the measuring apparatus, however, there is no inconvenience caused by taking a configuration including control units other than these control units. To be specific, the measurement control unit 20 includes a test strip checking unit 21, a blood sugar level measuring unit 22, a measured result recording unit 23 and a hypoglycemia alarm unit 24. The test strip checking unit 21 is a function unit that checks whether the test strip for measuring the blood sugar level described above is set on the side of the blood sugar level measuring apparatus 1 and is in a measurement-enabled status or not; and the blood sugar level measuring unit 22 is a function unit that measures the blood sugar level of the user on the basis of an output signal from the checked test strip. Furthermore, the measured result recording unit 23 is a function unit for saving and recording data of the measured result by the blood sugar level measuring unit 22 on a recording device (memory) within the blood sugar level measuring apparatus 1. Moreover, the hypoglycemia alarm unit 24 is a function unit that notifies the user of an alarm saying that the measured result indicates hypoglycemia if the measured result is lower than a reference range related to a proper blood sugar level.

Further, the management control unit 30 includes a condition flag setting unit 31, an elapse time setting unit 32 and a parameter setting unit 33. The condition flag setting unit 31 is a function unit that sets a flag related to a body condition that affects the blood sugar level of the user when measuring the blood sugar level, and, though an in-depth description thereof will be made later on, the condition flag setting unit 31 sets the condition flags about a post-exercise condition, a pre-meal condition and a post-meal condition in the first working example. In the first working example, the elapse time setting unit 32 is a function unit that sets elapse time since a point of time when the meal has started on such an occasion that the condition flag setting unit 31 sets the condition flag of the post-meal condition, and details thereof will be mentioned later on. Note that a starting point of this elapse time may be a predetermined point of time during the meal and a point of time when the meal has finished. Moreover, the parameter setting unit 33 is a function unit that sets a variety of control parameters used in the blood sugar level measuring apparatus 1. The parameters are exemplified by date/time used in the blood sugar level measuring apparatus 1 and a container unsealing date etc of the test strip. These function units possessed by the management control unit 30 enable the user to efficiently manage the measured result of the user's blood sugar level.

Further, the display control unit 40 includes a measurement graph display unit 41, a measurement average value display unit 42 and a measurement history display unit 43. The measurement graph display unit 41 is a function unit for displaying a graph of the measured results given by the measurement control unit 20. Moreover, the measurement average value display unit 42 is a function unit that displays an average value of the measurement values within a predetermined period in the measured results. Furthermore, the measurement history display unit 43 is a function unit that accesses the measured data recorded in the recording device of the blood sugar level measuring apparatus 1 and sequentially displays the measurement histories and even the detailed data thereof as the case may be. The displays of these function units possessed by the display control unit 40 are given on the liquid crystal screen 4 of the blood sugar level measuring apparatus 1 and enable the user to check transitions etc of the user' s blood sugar level via the screen display.

Further, the external communication unit 50 is the control unit that transmits especially the measured results of the measurement control unit 20 and the information on the condition flags etc managed by the management control unit 30 to the outside of the blood sugar level measuring apparatus 1. In the blood sugar level measuring apparatus 1 according to the present invention, the communication control of the external communication unit 50 is not the core element, and hence the present description restricts the discussion to the description that the external communication unit 50 exhibits the external communicating function, and an in-depth description thereof is omitted.

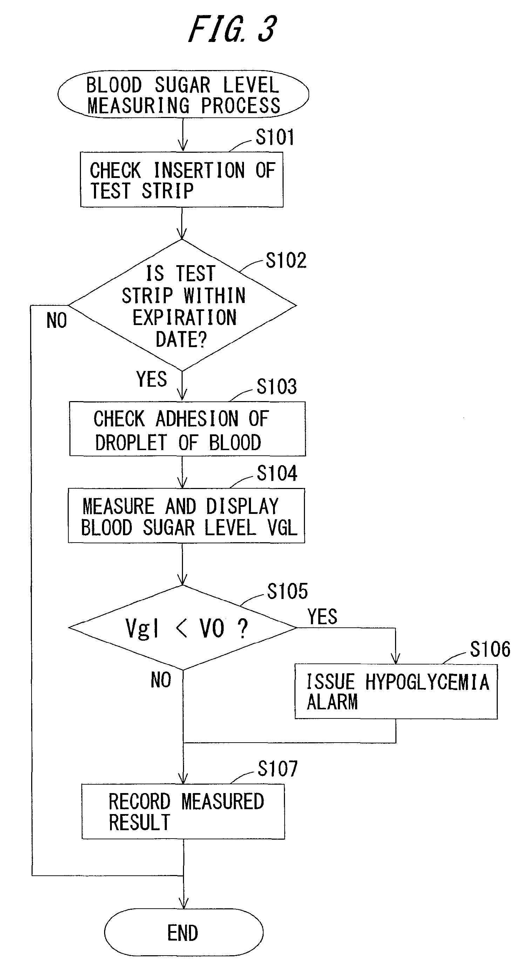

In the blood sugar level measuring apparatus 1, the variety of processes related to the measurements of the blood sugar levels of the user are realized by the measurement control unit 20, the management control unit 30 and the display control unit 40 respectively or by linking these respective control units with each other. Such being the case, hereafter, detailed contents of the processes will be described. At first, a blood sugar level measuring process executed by the measurement control unit 20 will be described based on FIG. 3. To start with, in S101, it is checked whether or not the test strip used for measuring the blood sugar level is inserted into the test strip insertion groove 10 of the blood sugar level measuring apparatus 1. Specifically, the test strip checking unit 21 determines based on a change in a resistance value etc in the test strip insertion groove 10 in a way that depends on whether the test strip is inserted or not. When the insertion of the test strip is confirmed in S101, processes from S102 onward are executed.

Similarly, the test strip checking unit 21 determines in S102 whether the test strip with its insertion being confirmed in S101 is within an expiration date or not. The test strip uses the GOD by way of oxidoreductase as described above, and therefore, if a fixed period elapses since the container stored with the test strip was unsealed, reactivity on the glucose in the blood declines with difficulty in properly measuring the blood sugar level, whereby it is preferable that the test strip is used for the measurement within the fixed period after being unsealed. Then, for instance, when unsealing a bottle hermetically containing a plurality of test strips, the user inputs unsealing date/time to the blood sugar level measuring apparatus 1 by operating the operation button 7, and the parameter setting unit 33 of the management control unit 30 sets, as an expiration date of the test strip, date/time after, e.g., six months since the unsealing date/time has been inputted. Accordingly, it follows that the determination in S102 is made based on whether the date/time when measured exceeds the thus-set expiration date or not. If determined to be affirmative in S102, the processing advances to S103, in which preparations for the measurement are made. Whereas if determined to be negative in S102, an attention of the user is evoked by saying that the test strip itself is not suited to the measurement of the blood sugar level, or alternatively the present blood sugar level measuring process is finished.

In S103, the blood sugar level measuring unit 22 checks whether or not a droplet of blood is adhered to the test strip. To be specific, the checking of whether the droplet of blood is adhered or not is conducted based on an output current from the sensor within the test strip, which is generated by the user's adhering the droplet of self-blood onto the test strip. The blood sugar level measuring unit 22 executes, with the confirmation that the droplet of blood is adhered, the predetermined processes for measuring the blood sugar level and finally measures a blood sugar level Vgl, and the measured result is displayed on the liquid crystal screen 4 (process in S104). Note that the technology of measuring the blood sugar level of the droplet of blood adhered onto the test strip is of the prior art, and hence an in-depth description thereof is omitted in the present description.

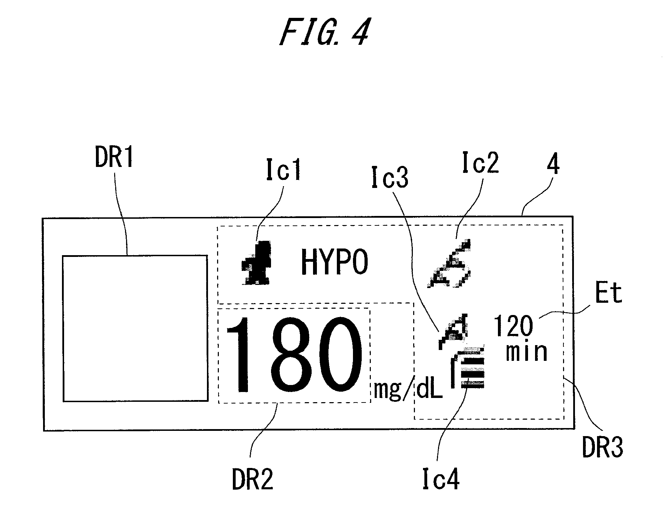

Herein, FIG. 4 depicts one example of the display on the liquid crystal screen 4. The example of the display screen depicted in FIG. 4 is that icons and numerical values are displayed in aggregation for the sake of the explanation. A graph display region DR1, i.e., a rectangular display region, for displaying a graph that will be described later on is formed on the left side of the liquid crystal screen 4, a measured numerical value display region DR2 for displaying the measured blood sugar level etc is formed at the central portion thereof, and an icon display region DR3 of displaying icons having a variety of meanings and a predetermined period of elapse time is formed in the periphery of the measured numerical value display region DR2. The icons displayed in this icon display region DR3 include an icon "HYPO" for an alarm against the hypoglycemia displayed in S106 that will be described later on, an icon Ic1 for a post-exercise flag serving as the condition flag illustrated in FIG. 4, an icon Ic2 for a pre-meal flag, a first icon Ic3 for a post-meal flag and a second icon Ic4 for the post-meal flag. Further, a post-meal elapse time Et is displayed adjacent to the first and second icons for the post-meal flag in the icon display region DR3. These icons for the condition flags and the post-meal elapse time Et will be described later on. Note that the graph display region DR1 is configured as a dot indicator and is configured to enable free forms to be displayed properly. The icon display region DR3 is configured to enable predetermined icon shapes to be displayed by a lighting instruction of the condition flag setting unit 31. Furthermore, the blood sugar level measuring apparatus 1 includes a memory stored with information on display objects (graphs and flags) in the graph display region DR1, and the condition flag setting unit 31 can properly read the information on the predetermined flag from within the flags stored in the memory and display the readout information in the graph display region DR1 in the way of being associated with corresponding items of measurement data.

Next, it is determined in S105 whether the blood sugar level Vgl measured in S104 is lower than a reference blood sugar level V0 or not. This reference blood sugar level V0 is a threshold value for determining the hypoglycemia and is a value that is preset on the side of the blood sugar level measuring apparatus 1. Then, if determined to be affirmative in S105, the processing advances to S106, in which the hypoglycemia alarm unit 24 notifies the user of the alarm indicating the hypoglycemia. To be specific, the characters "HYPO" are lit up together with the measured blood sugar level on the liquid crystal screen 4, thereby giving the notification of the hypoglycemia alarm. Moreover, whereas if determined to be negative in S105 or when the process in S106 is finished, the processing advances to S107. In S107, the measured result recording unit 23 records a value of the measured blood sugar level as the measured result on a recording device in the blood sugar level measuring apparatus 1. The measured result obtained by the blood sugar level measuring process is used for a measured result management process, a graph display process and a measurement history display process that will be described later on.

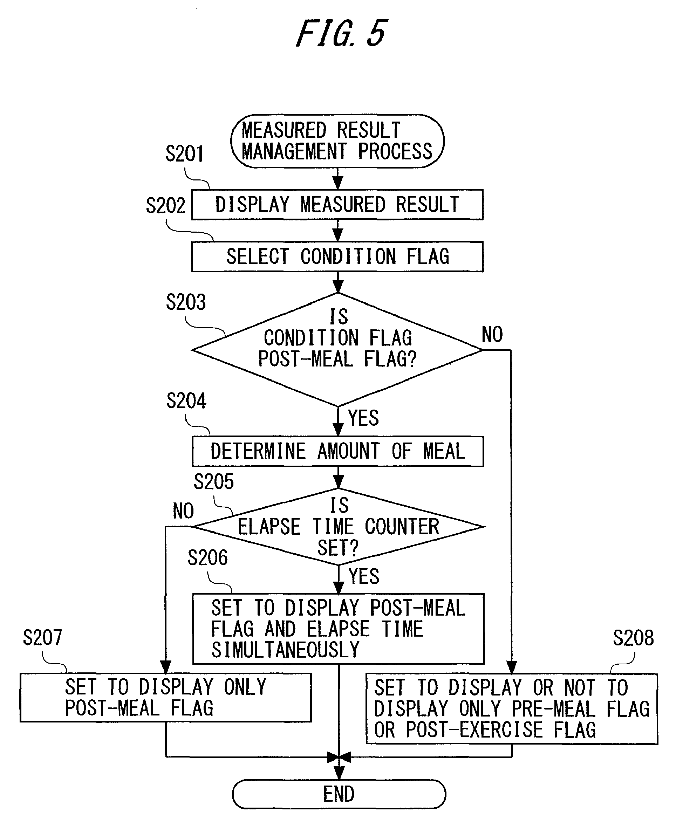

Next, the measured result management process executed by the condition flag setting unit 31 of the management control unit 30 will be described based on FIG. 5. The measured result management process is defined as control for managing the measured result obtained by the blood sugar level measuring process. At first, in S201, the measurement value, i.e., the measured result is displayed in the measured numerical value display region DR2 on the liquid crystal screen 4 immediately after executing the blood sugar level measuring process. Incidentally, an assumption at this time is that any icons for the condition flags such as the icon for the post-exercise flag described above are not displayed in the icon display region DR3. Upon finishing the process in S201, the processing advances to S202.

In S202, in the status where the measurement value is displayed, when the user operates the operation button 7 to perform the displayed measurement, the condition flag corresponding to a condition of the user's body is selected. Specifically, the user selects, as the condition flag, any one of four flags, i.e., the post-exercise flag, the pre-meal flag, the post-meal flag and no condition flag, and it follows that the condition flag corresponding to this selection is recorded in the way of being associated with the displayed measurement value in the blood sugar level measuring apparatus 1. These condition flags are flags related to the conditions of the user's body that have a strong relationship with the blood sugar level of the user. It is therefore highly useful in terms of managing the blood sugar level that the condition flag is associated with the-thus measured blood sugar level. Note that these condition flags respectively correspond to the icon Ic1 for the post-exercise flag, the icon Ic2 for the pre-meal flag, the first and second icons Ic3, Ic4 for the post-meal flag and non-display of icon illustrated in FIG. 4, whereby it follows that the icons are displayed on the liquid crystal screen 4. The icon Ic2 for the pre-meal flag is the icon taking a shape imitating a long and thin bread loaf that is kept intact as a whole, and the first icon Ic3 for the post-meal flag is the icon taking a shape imitating a bread loaf of which a part is eaten and shortened in length. Thus, correlativeness is given to the shape of the icon, whereby the user distinguishes at a glance between the icon for the pre-meal flag and the icon for the post-meal flag. Note that the shapes of the icons representing the pre- and post-meal conditions may not be those bread loafs, and other various forms can be adopted. Upon finishing the process in S202, the processing advances to S203.

It is determined in S203 whether the condition flag selected in S202 is the post-meal flag or not. Herein, the processing advances to S204 if determined to be affirmative but diverts to S208 whereas if determined to be negative.

In S204, an amount of meal corresponding to the post-meal flag is determined. Through this process, additional information about how much amount of meal the user has taken at the measurement can be associated with the post-meal flag set by the blood sugar level measuring apparatus 1. To be specific, for example, the user can set the amount of meal selectively from three types of percentages such as 50%, 80% and 100% if 100% is set based on user's subjectivity when satiated with the meal. Note that in the blood sugar level measuring apparatus 1 according to the first working example, this selection of the post-meal flag is indicated by displaying the first icon Ic3 for the post-meal flag in the icon display region DR3 in FIG. 4, while the amount of meal is indicated together with displaying the second icon Ic4 for the post-meal flag in a way that changes the number of bars displayed within the icon. For example, the case of 100% as the amount of meal is indicated by three lengths of bars (a status depicted in FIG. 4), the case of 80% is indicated by two bars, and the case of 50% is indicated by one bar, respectively. Thus, the data on the amount of meal is associated with the post-meal flag, thereby enabling the user to know how much amount of meal the user has taken when the blood sugar level is measured and facilitating the understanding of a relation between the amount of meal and the blood sugar level and management of health in relation to the blood sugar level by adjusting the amount of meal, etc. It is to be noted that the second icon Ic4 with respect to the display of the amount of meal takes a shape imitating the stomach of a person. Then, one to three bars are displayed within the stomach, whereby the user can easily recall the "amount of meal" simply by glancing at the icon displayed therein. Further, the second icon Ic4 is not limited to the shape imitating the stomach of the person.

It is determined in S205 whether an elapse time counter is set in the blood sugar level measuring apparatus 1 or not. The elapse time counter is a function set by the elapse time setting unit 32 of the blood sugar level measuring apparatus 1 in advance of the measurement and is also a function of automatically counting the elapse time since a point of time when starting having the meal and associating this elapse time with the blood sugar level of the measured result. Note that this elapse time is displayed as the post-meal elapse time Et in the icon display region DR3 in FIG. 4. With this contrivance, the user can recognize, in addition to the amount of meal, how much time has elapsed since the start of having the meal when the blood sugar level is measured, and gets therefore easier to conduct the health management in relation to the blood sugar level. The processing advances to S206 if determined to be affirmative in S205 but advances to S207 if determined to be negative.

The setting is done in S206 so that the post-meal flag and the post-meal elapse time Et are simultaneously displayed on the liquid crystal screen 4. With respect to the display of this post-meal flag, as described above, the first icon Ic3 and the second icon Ic4 for the post-meal flag come to the display status, and the bar count corresponding to the amount of meal is set in the second icon Ic4. On the other hand, the setting is done in S207 so that only the post-meal flag is displayed without displaying the post-meal elapse time Et.

Moreover, when the processing advances to S208 if determined to be negative in S203, the setting is done in S208 on the liquid crystal screen 4 so as to display the icon corresponding to any one of the pre-meal flag, the post-exercise flag and non-selection of the flag in the condition flags selected in S202. Specifically, when the pre-meal flag and the post-exercise flag are selected, it follows that the icon Ic2 for the pre-meal flag and the icon Ic1 for the post-exercise flag are respectively displayed in the icon display region DR3. If the flag is in the non-selection status, any icon corresponding to the condition flag is not displayed in the icon display region DR3.

As described above, according to the measured result management process, the data can be retained in the blood sugar level measuring apparatus 1 by associating the measured blood sugar level with the condition flag related to the body condition of the user when measured, thereby facilitating the health management pertaining to the blood sugar level of the user. Especially also in a graph display process and a measurement history display process that will be described later on, the data useful for the health management using the blood sugar level measuring apparatus 1 can be displayed by making use of the condition flags associated with the measured blood sugar levels.

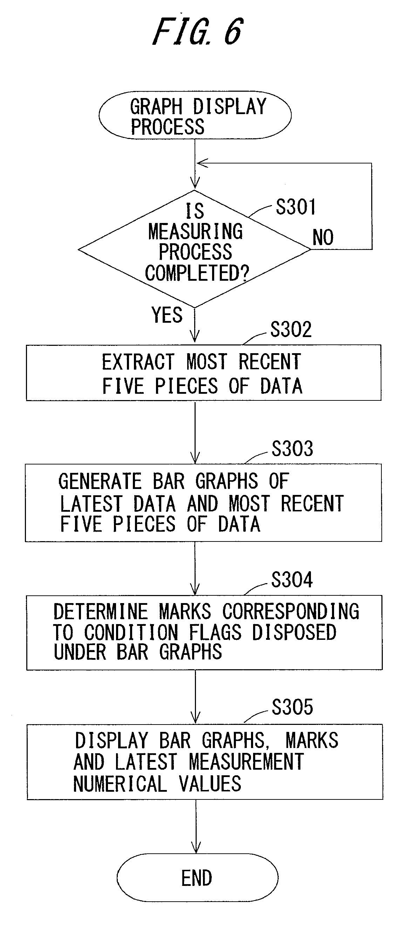

Next, the graph display process executed by the measurement graph display unit 41 of the management control unit 30 will be described based on FIG. 6. The graph display process is defined as control for displaying the measured result obtained by the blood sugar level measuring process. To start with, it is determined in S301 whether the measurement of the blood sugar level in the blood sugar level measuring process is completed or not. If determined to be affirmative, the processing advances to S302, but, if determined to be negative, the process in S301 is again executed. In S302, the data of the most recent measurements for five times, which have already been acquired and recorded in the recording device of the blood sugar level measuring apparatus 1, are extracted separately from the latest data obtained by the completed blood sugar level measuring process. Upon finishing the process in S302, the processing advances to S303.

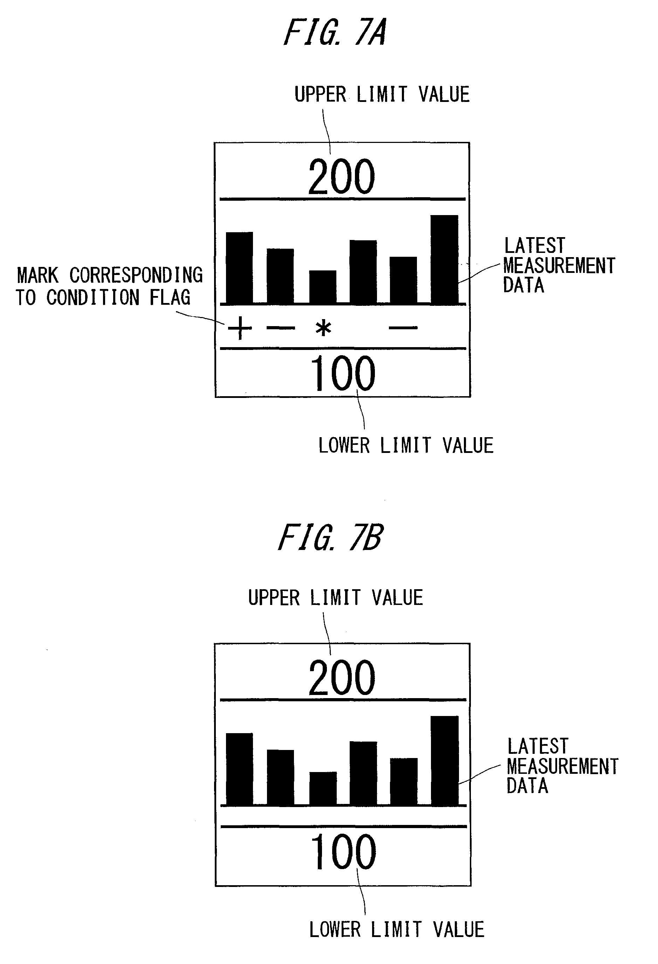

In S303, a bar graph is generated based on the latest data acquired by the blood sugar level measuring process and the most recent data for five times that are extracted in S302 in order to display the respective measured blood sugar levels in the form of the bar graph on the liquid crystal screen 4. Herein, the blood sugar level measuring apparatus is a portable apparatus, and therefore the liquid crystal screen 4 is comparatively small in size. Hence, a scheme is not that the bar graph is generated by making a size of the bar graph proportional to a magnitude itself of the measured blood sugar level but that the bar graph is displayed between a preset upper limit value and a preset lower limit value. With this scheme, the user gets easy to recognize a change per measurement of the blood sugar level even on the relatively small liquid crystal screen 4. Note that the bar graph generated in S303 is displayed in the graph display region DR1 depicted in FIG. 4, and FIG. 7A illustrates a display example thereof. In FIG. 7A, a numerical value displayed on the upper side is the upper limit value, while a numerical value displayed on the lower side is the lower limit value. When finishing the process in S303, the processing advances to S304.

In S304, if the condition flags are attached to the most recent data for five times that are extracted in S302, a mark (symbol) corresponding to the type of the condition flag is determined in order to display this mark under the bar graph of each data. In the blood sugar level measuring apparatus 1 of the first working example, when the post-exercise flag, the pre-meal flag and the post-meal flag are selected as the condition flags, these flags are selected so that marks such as an asterisk (*) mark, a minus (-) mark and a plus (+) mark are arranged. Incidentally, if the condition flag is not selected, the display mark is not selected in S304. Upon finishing the process in S304, the processing advances to S305.

In S305, the bar graph generated in S303 and a screen (e.g., the screen illustrated in FIG. 7A) configured to include the marks determined in S304 are displayed in the graph display region DR1, while the latest numerical value of the measured blood sugar level, which is set as a target of the determination of the completion in S301, is displayed in the measured numerical value display region DR2. Note that the latest measured result may undergo the measured result management process in FIG. 5 in this status.

Further, FIG. 7B depicts another form of the bar graph displayed in the graph display region DR1. In the form depicted in FIG. 7B, the illustrations of the marks (symbols) corresponding to the types of the condition flags in the graphs depicted in FIG. 7A are omitted. Even this display form enables the user to confirm the transitions of the blood sugar levels and is therefore useful for the management of the blood sugar level, which is the same as the previous display form.

According to the graph display process, the data of the blood sugar levels measured in the past are displayed by the bar graphs, and besides the marks corresponding to the condition flags are also displayed, whereby the user can easily recognize the body condition when measuring the blood sugar level to be measured together with the transitions of the blood sugar levels. Therefore, the health management related to the blood sugar level can be easily conducted. Further, as illustrated in FIG. 7, the information on the condition flag is expressed by using the simple mark, and it is therefore feasible to surely transfer the information to the user even on the comparatively small liquid crystal screen 4.

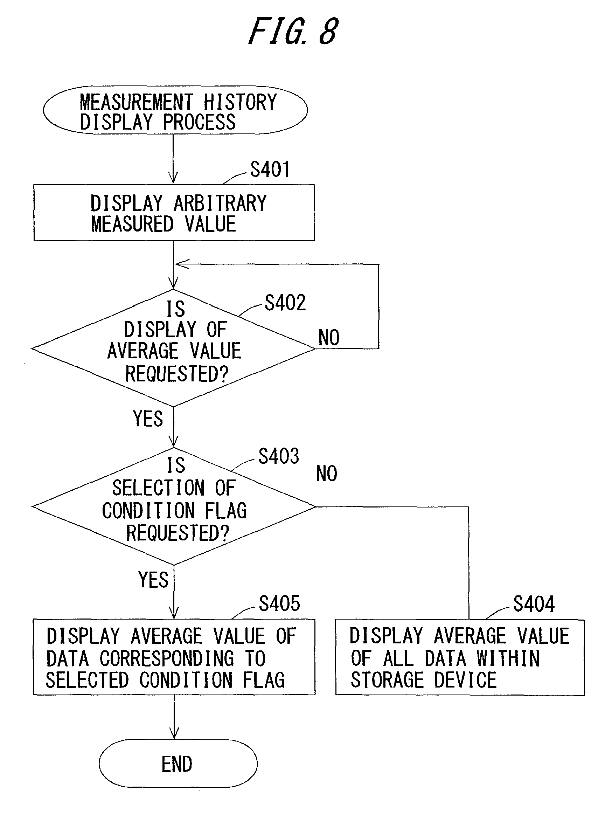

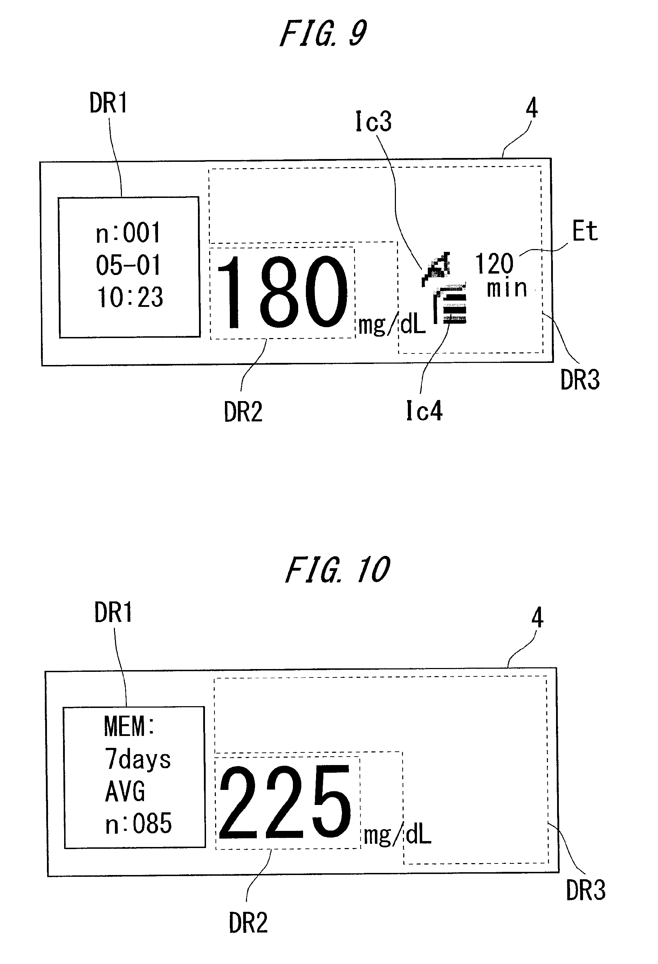

Next, the measurement history display process executed by the measurement average value display unit 42 and the measurement history display unit 43 of the display control unit 40, will be described based on FIG. 8. The measurement history display process is defined as control for displaying a history of the measured results obtained by the blood sugar level measuring process. To begin with, in a power-ON status of the blood sugar level measuring apparatus 1, in S401, an arbitrary measurement value taken from within the measurement results recorded on the recording device of the blood sugar level measuring apparatus 1 is displayed on the liquid crystal screen 4, and a display example thereof is depicted in FIG. 9. The process in S401 is executed by the measurement history display unit 43. At first in S401, the latest measured result is automatically displayed, however, the user operates the operation button 7 by sliding this button, whereby the arbitrary measured result in the midst of being recorded can be displayed. Note that in this display process, measurement date/time (10:23, May 1 in the first working example) is displayed together with an identification number (n:001 in the first working example) of the measured result in the graph display region DR1. Further, the measured blood sugar level is displayed in the measured numerical value display region DR2, and, if the condition flag has been already set, the icon (the first and second icons Ic3, Ic4 for the post-meal flag in the first working example) corresponding to this condition flag and the post-meal elapse time Et are displayed in the icon display region DR3.

Next, the processes from S402 onward will be described. The processes from S402 onward are the processes that are executed by the measurement average value display unit 42. It is determined in S402 whether an average value display request is given from the user or not. Concretely, it is determined based on the user' s operation of the operation button 7 whether the average value display request is given or not. Herein, if determined to be affirmative, the processing advances to S403, and, whereas if determined to be negative, the processes from S402 onward are again executed. It is determined in S403 whether a request for selecting the condition flag in displaying the average value is given from the user or not. To be specific, it is determined based on the user's operation of the operation button 7 whether the request for selecting the condition flag in displaying the average value is given from the user or not. Herein, the processing advances to S405 if determined to be affirmative and advances to S404 whereas if determined to be negative.

In S404, an average value of the data of all the measured blood sugar levels recorded on the recording device is displayed, and a display example thereof is illustrated in FIG. 10. In this display process, a period (which is a period of 7 days in the first working example. This period is a period tracing back to the past on the basis of the date/time when executing the present measurement history display process.) for calculating the average value and, together with this period, a population parameter (n:085 in the first working example) of the average value are displayed in the graph display region DR1 . Accordingly, the average value of the blood sugar levels of a measurement count "85" for 7 days is displayed in the measured numerical value display region DR2. On the other hand, the icon etc corresponding to the condition flag is not displayed in the icon display region DR3.

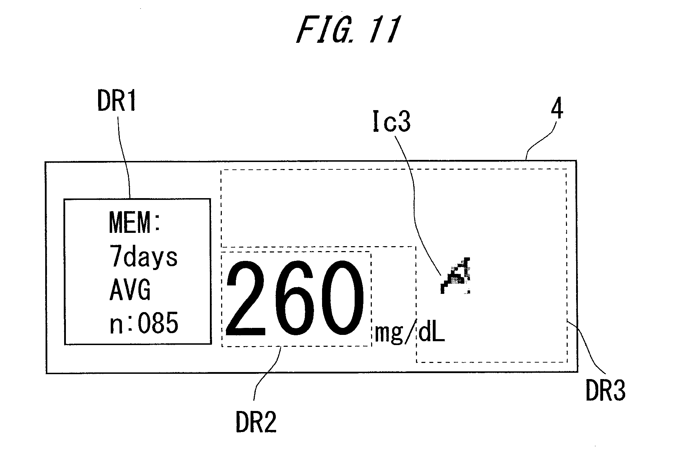

Next, in S405, the data corresponding to the condition flags requested by the user to be selected in the data of all the measured blood sugar levels recorded on the recording device are extracted, then an average value of the extracted data is calculated and displayed, and a display example thereof is depicted in FIG. 11. That is, the average value displayed in S404 is, irrespective of the type of the condition flag and of whether there is the condition flag or not, the average value of all the measured results measured for the period of 7 days, however, the average value displayed in S405 is equivalent to an average value given when the user considers further narrowing down the average value per condition flag from within the measured results becoming the processing target in S404. Accordingly, the user can get each average value to be displayed per post-exercise measured result, pre-meal measured result and post-meal measured result respectively from within the measured results for, e.g., 7 days.

Note that in the display process of FIG. 11 in the first working example, the user selects the post-meal flag as the condition flag, and hence the first icon Ic3 in the icons for the post-meal flag is displayed. It is to be noted that the calculation of the average value corresponding to the post-meal flag does not involve making a distinction based on the amount of meal, so that the second icon Ic4 in the icons for the post-meal flag is in the non-display status. Then, the population parameter (n:025 in the first working example) of the measured blood sugar level associated with the post-meal flag is displayed together with the period (7 days in the first working example) for calculating the average value are displayed in the graph display region DR1. Therefore, the average value of the blood sugar levels of the measurement count "25" for 7days is displayed in the measured numerical value display region DR2.

When finishing the process in S404 or S405, the measurement history display process comes to an end. Herein, in the measurement history display process, a freely switchable formation between the process in S404 and the process in S405 is also available. That is, the display of the average value of the blood sugar level corresponding to the condition flag selected in S404 and the display of the average value of the data of all the blood sugar levels in the recording device are set properly switchable by the user' s operation, thereby further improving the convenience of the blood sugar level measuring apparatus 1.

According to the measurement history display process, the user can confirm, together with the condition flags, the arbitrary history of the measured results recorded in the blood sugar level measuring apparatus 1. Further, the average value of the past measured results can be confirmed separately per condition flag, and therefore the health management related to the blood sugar level can be easily conducted. Note that the period for calculating the average value can be also changed to an arbitrary period via the parameter setting unit 33.

Note that an alarm function of notifying the user of the time when measuring the blood sugar level may be incorporated into the blood sugar level measuring apparatus 1 according to the present invention. The user presets the measurement time, and a notification sound is thereby emitted just when reaching the preset time, thus making it possible to prevent the user from forgetting the measurement. Furthermore, if the user does not operate the apparatus for a fixed period of time in terms of restraining power consumption of the blood sugar level measuring apparatus 1, the blood sugar level measuring apparatus 1 may incorporate such a power consumption restraining function that the power source of the apparatus automatically comes into an OFF status, or an intensity of display illumination of the liquid crystal screen 4 is automatically decreased (darkened). The power consumption restraining function such as this is useful particularly for the blood sugar level measuring apparatus 1 as the portable apparatus.

### [Second Working Example]

### <Other Working Examples>

The present invention includes, as described above, a computer program for executing the blood sugar level measuring process, the measured result management process, the graph display process and the measurement history display process by way of the embodiment. Moreover, such a type of medium that this program is recorded on a computer readable recording medium also belongs to the category of the present invention. The computer is made to read and execute the program recorded on the recording medium, thereby enabling those respective processes to be carried out.

Herein, the computer readable recording medium connotes a recording medium capable of accumulating information such as data and programs electrically, magnetically, optically, mechanically or by chemical action, which can be read from the computer. Among these recording mediums, for example, a floppy (registered trademark) disk, a magneto-optic disc, a CD-ROM, a CD-R/W, a DVD, a Blu-ray disc, a DAT, an 8 mm tape, a memory card, etc. are given as those removable from the computer. Further, a hard disc, a ROM (Read-Only Memory), etc. are given as the recording mediums fixed within the computer.

### [Third Working Example]

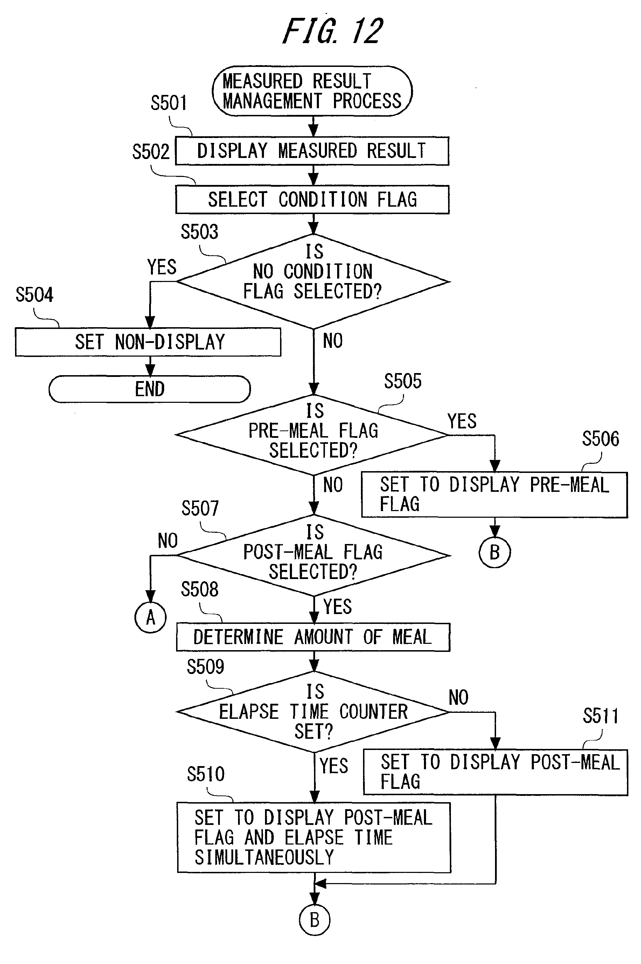

Next, the measured result management process executed by the condition flag setting unit 31 of the management control unit 30 according to a third working example will be described based on FIGS. 12 through 19. The condition flag setting unit 31 of the management control unit 30 according to the third working example is a function unit that sets the flag pertaining to the body condition affecting the blood sugar level of the user on the occasion of measuring the blood sugar level, and sets the condition flags such as a pre-meal flag, a post-meal flag, a post-exercise flag, a post-insulin-dosing flag, a β-cell flag, a cholesterol flag and a Hb (haemoglobin) A1c flag. These flags may be fixedly set on the liquid crystal screen 4 so that predetermined icons are lit up in the icon display region DR3, and pieces of information on the icon shapes may also be stored on the memory so that the predetermined icons are displayed in the graph display region DR1. The information on the icon shapes may be stored on the memory when manufacturing the product, and, for instance, after manufacturing the product, changes (addition, deletion, etc) of information may also be applied to the information stored when manufactured. The information on the icon shapes may be stored by, e.g., manually inputting predetermined information to the memory in a way that uses such as the operation button 7 of the blood sugar level measuring apparatus 1 and a separate input means or by automatically inputting the predetermined information (e.g., the program etc) to the memory wirelessly or via the cable from an external device (e.g., an external PC and a server). Note that the condition flag setting unit 31 may, without being limited to the examples given above, set whatever flags representing phenomena and events related to the body conditions. The third working example will discuss the control for the measured result management process to manage the measured results obtained by the blood sugar level measuring process by way of an exemplification. At first, in S501, immediately after executing the blood sugar level measuring process, the measurement value as the measured result is displayed in the measured numerical value display region DR2 on the liquid crystal screen 4. Incidentally, it is assumed at this time that any icon for the condition flag such as the icon for the post-exercise flag is not displayed in the icon display region DR3. Upon finishing the process in S501, the processing advances to S502.

In S502, in the status where the measurement value is displayed, on the occasion of making the measurement displayed therein, the user selects the condition flag corresponding to the body condition of the user by operating the operation button 7. To be specific, the user selects, as the condition flag(s), any one or more flags from within the pre-meal flag, the post-meal flag, the post-exercise flag, the post-insulin-dosing flag, the β-cell flag, the cholesterol flag, the HbA1C flag and no condition flag.

The user can make the selection about the amount of meal in the case of choosing the post-meal flag. Further, the user may input the amount of meal when choosing the post-meal flag. The user can make the selection concerning the amount of exercise in the case of selecting the post-exercise flag. The user may input the amount of exercise when selecting the post-exercise flag. The user can make the selection about the amount of insulin dosage in the case of choosing the post-insulin-dosing flag. The user may input the insulin dosage when selecting the post-insulin-dosing flag. The user can make the selections related to an amount of β cells and a change in amount of β cells in the case of choosing the β-cell flag. The user may input the amount of β cells and the change in amount of β cells when choosing the β-cell flag. The user can make the selections pertaining to an amount of cholesterol and a change in amount of cholesterol in the case of choosing the cholesterol flag. The user may input the amount of cholesterol and the change in amount of cholesterol when choosing the cholesterol flag. The user can make the selections about a HbA1C value and a change in HbA1C value in the case of choosing the HbA1C flag. The user may input the HbA1C value and the change in HbA1C value when selecting the HbA1C flag.

An operating example of the operation button 7 by the user is that the user operates the operation button 7 by sliding this button, with the result that the icons corresponding to the respective flags displayed on the liquid crystal screen 4 and the marks indicating the amounts are sequentially changed over, and the user presses the operation button 7, with the result that the flags are selected. In the blood sugar level measuring apparatus 1 according to the third working example, to start with, the user operates the operation button 7 so as to slide the button, thereby displaying the icon corresponding to the pre-meal flag on the liquid crystal screen 4. In the status where the icon corresponding to the pre-meal flag is displayed on the liquid crystal screen 4 when the user performs the slide operation of the operation button 7, a changeover to the icon corresponding to the pre-meal flag is made, and the icon corresponding to the post-meal flag is displayed on the liquid crystal screen 4. Then, in the status where the icon corresponding to the post-meal flag is displayed on the liquid crystal screen 4, when the user conducts the slide-operation of the operation button 7, the icon corresponding to the post-meal flag and the mark indicating the amount of meal are displayed on the liquid crystal screen 4. The mark indicating the amount of meal is the bar mark, in which an increase or a decrease in amount of meal is changed by incrementing or decrementing the number of bar marks. Specifically, the user performs the slide operation of the operation button 7, whereby a set of the icon corresponding to the post-meal flag and one length of bar mark, another set of the icon corresponding to the post-meal flag and two lengths of bar marks and still another set of the icon corresponding to the post-meal flag and three lengths of bar marks are displayed in the way of being sequentially changed over on the liquid crystal screen 4. The mark indicating the amount of meal is not, however, limited to the bar mark, and other types of marks are also available.

Then, in the status where the icon corresponding to the post-meal flag and the mark indicating the amount of meal are displayed on the liquid crystal screen 4, when the user carries out the slide operation of the operation button 7, the display is changed over from the icon corresponding to the post-meal flag and the mark indicating the amount of meal, and the icon corresponding to the post-exercise flag is displayed on the liquid crystal screen 4. Subsequently, in the status where the icon corresponding to the post-exercise flag is displayed on the liquid crystal screen 4, when the user performs the slide operation of the operation button 7, the icon corresponding to the post-exercise flag and the mark indicating the amount of exercise are displayed on the liquid crystal screen 4. Also with respect to the icon corresponding to the post-exercise flag and the mark indicating the amount of exercise, similarly the icon corresponding to the post-meal flag and the mark indicating the amount of meal, the user conducts the slide operation of the operation button 7, thereby changing the increase or the decrease in amount of exercise. Also in regard to a set of the icon corresponding to the insulin-dosing flag and the mark indicating the amount of insulin dosage, a set of the icon corresponding to the β-cell flag and the mark indicating the amount of β cells, a set of the icon corresponding to the cholesterol flag and the mark indicating the cholesterol value and a set of the icon corresponding to the HbA1C flag and the mark indicating the HbA1C value, the operations are the same as those with respect to the set of icon corresponding to the post-meal flag and the mark indicating the amount of meal. In the status where any icon is not displayed on the liquid crystal screen 4, when the user presses the operation button 7, no condition flag is selected. After the flag has been selected, the user removes the test strip from the test strip insertion groove 10, thereby establishing the selection of the flag.

The condition flag is the flag related to the body condition having the strong relationship with the blood sugar level of the user. It is therefore highly useful in terms of managing the blood sugar level to associate the thus-measured blood sugar level with the condition flag. Note that the post-exercise flag, the pre-exercise flag and the post-meal flag correspond to the icon Ic1 for the post-exercise flag, the icon Ic2 for the pre-meal flag, the first and second icons Ic3, Ic4 for the post-meal flag and non-display of the icon respectively, which are displayed on the liquid crystal screen 4 as well as being illustrated in FIG. 4. The icon Ic2 for the pre-meal flag is the icon taking the shape imitating the long and thin bread loaf that is kept intact as a whole, and the first icon Ic3 for the post-meal flag is the icon taking the shape imitating the bread loaf of which a part is eaten and shortened in length. Thus, the correlativeness is given to the shape of the icon, whereby the user distinguishes at a glance between the icon for the pre-meal flag and the icon for the post-meal flag. Note that the shapes of the icons representing the pre- and post-meal conditions may not be those bread loafs, and other various forms can be adopted. The icons corresponding to the post-insulin-dosing flag, the β-cell flag, the cholesterol flag and the HbA1C flag are displayed in the graph display region DR1 depicted in FIG. 4, and the details thereof will be described later on. Upon finishing the process in S502, the processing advances to S503.

It is determined in S503 whether no condition flag is selected in S502 or not. Herein, the processing diverts to S504 if determined to be affirmative but advances to S505 whereas if determined to be negative.

The setting on the liquid crystal screen 4 is done in S504 so that any icons corresponding to the condition flags are not displayed in the graph display region DR1 and the icon display region DR3 depicted in FIG. 4. The flag is in the non-selection status in S502, and hence the icon corresponding to the condition flag is not displayed.

It is determined in S505 whether the pre-meal flag is selected in S502 or not. Herein, the processing diverts to S506 if determined to be affirmative but advances to S507 whereas if determined to be negative.

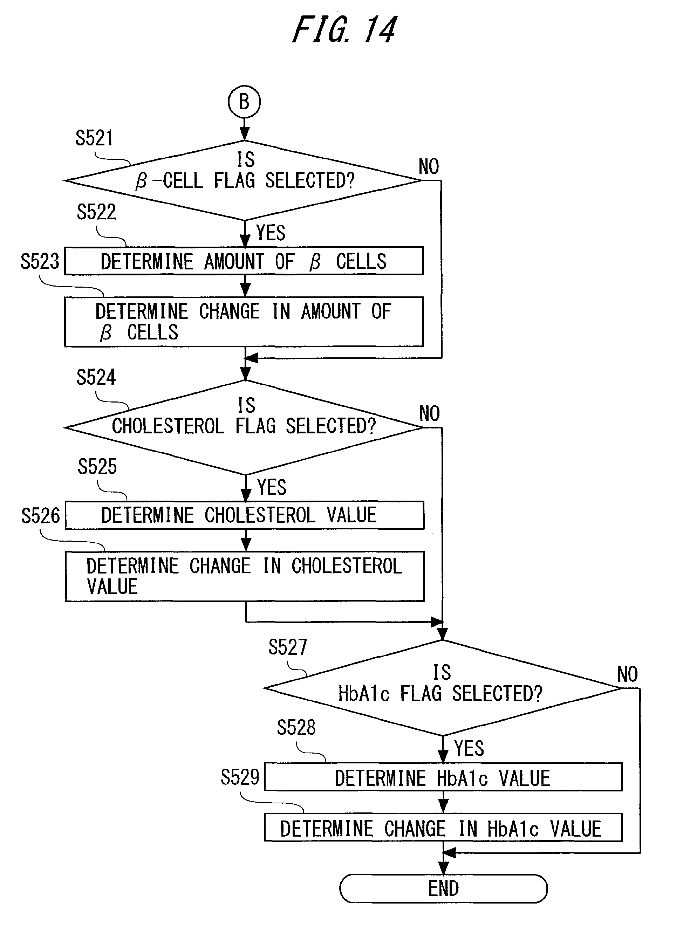

The setting on the liquid crystal screen 4 is done in S506 so that the icon corresponding to the pre-meal flag selected in S502 is displayed. Concretely, the setting is done so that the icon Ic2 for the pre-meal flag is displayed in the icon display region DR3. Upon an end of S506, the processing shifts to S521 (FIG. 14). Processes from S521 onward will be described later on.

It is determined in S507 whether the post-meal flag is selected in S502 or not. Herein, the processing advances to S508 if determined to be affirmative but diverts to S511A whereas if determined to be negative. Processes from S511A onward will be described later on.

In S508, the amount of meal corresponding to the post-meal flag is determined. The process executed in S508 is the same as the process carried out in S204 of the measured result management process explained in the first working example, and therefore its description is omitted. If an amount of ingested energy is inputted as the amount of meal in S502, the amount of ingested energy may also be determined as the amount of meal corresponding to the post-meal flag. Then, the amount of ingested energy determined as the amount of meal is recorded on the recording device within the blood sugar level measuring apparatus 1. Note that in the process in S401 of the measurement history display process explained in the first working example, the user performs the slide operation of the operation button 7, whereby the amount of ingested energy recorded on the recording device within the blood sugar level measuring apparatus 1 is displayed on the liquid crystal screen 4. Concretely, an amount of consumption energy is displayed together with the identification number and the measurement date/time of the measured result in the graph display region DR1. When finishing S508, the processing advances to S509.

It is determined in S509 whether the elapse time counter is set in the blood sugar level measuring apparatus 1 or not. The process executed in S509 is the same as the process carried out in S205 of the measured result management process explained in the first working example, and hence the description thereof is omitted. The processing advances to S510 if determined to be affirmative in S509 but diverts to S511 whereas if determined to be negative.

The setting is done in S510 so that the icon of the post-meal flag and the post-meal elapse time Et are simultaneously displayed on the liquid crystal screen 4. To be specific, the setting is done so that the first icon Ic3 and the second icon Ic4 for the post-meal flag and the post-meal elapse time Et are displayed in the icon display region DR3. The number of bars within the second icon Ic4 for the post-meal flag is set corresponding to the amount of meal. Upon an end of S510, the processing advances to S521. On the other hand, the setting is done in S511 so that the post-meal elapse time Et is not displayed on the liquid crystal screen 4, while the first icon Ic3 and the second icon Ic4 for the post-meal flag are displayed in the icon display region DR3. The number of bars within the second icon Ic4 for the post-meal flag is set corresponding to the amount of meal. Upon an end of S511, the processing advances to S521.

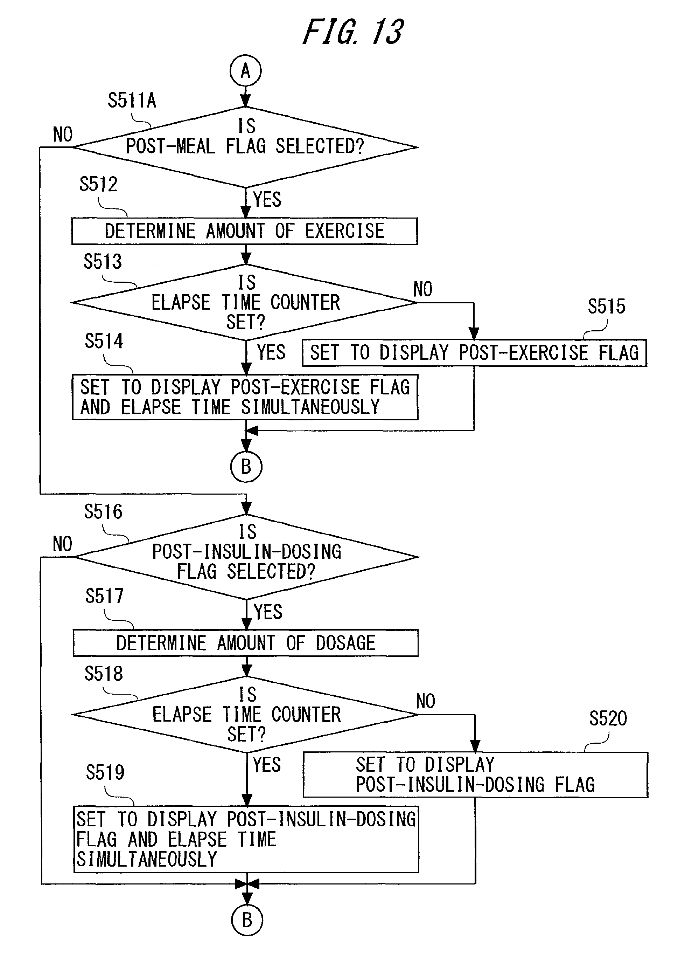

Processes from S511A (FIG. 13) onward will be described. It is determined in S511A whether the post-exercise flag is selected or not. Herein, the processing advances to S512 if determined to be affirmative but diverts to S516 whereas if determined to be negative.

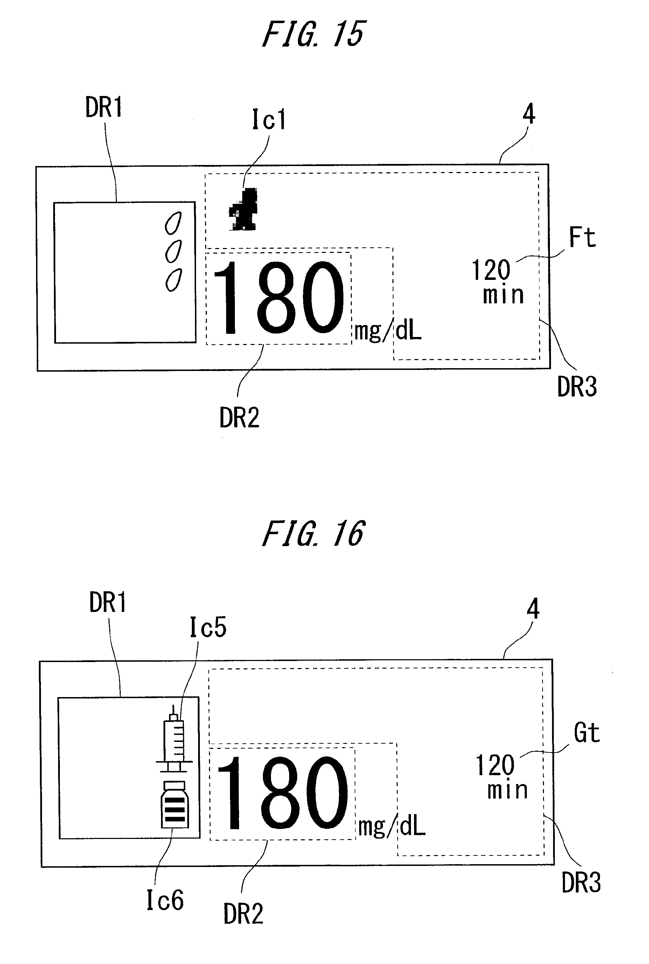

In S512, the amount of exercise corresponding to the post-exercise flag is determined. Through this process, the accompanying data on how much amount of exercise the user has done when making the measurement can be associated with the post-exercise flag set by the blood sugar level measuring apparatus 1. For example, the user can set the amount of exercise selectively from three types such as 100% given when taking the exercise for 60 min or longer, 80% given when taking the exercise for 30 min or longer but within 60 min and 50% given when taking the exercise within 30 min. The post-exercise flag is selected, as illustrated in FIG. 15, in the status where the icon Ic1 for the post-exercise flag is displayed in the icon display region DR3. Then, the amount of exercise is selected in the status where the icon imitating a sweat is, as illustrated in FIG. 15, displayed in the graph display region DR1 in a way that changes the number of icons imitating the sweats. For instance, if the amount of exercise is 100%, the number of icons imitating the sweats is "3" (which is the status depicted in FIG. 15), and if being 80% and 50%, the numbers of icons imitating the sweats are "2" and "1", respectively. Thus, the data about the amount of exercise is associated with the post-exercise flag, thereby enabling the user to know how much amount of exercise the user has taken when measuring the blood sugar level and to understand the relation between the amount of exercise and the blood sugar level and facilitating the health management related to the blood sugar level. The icons imitating one to three drops of sweats are displayed in the graph display region DR1, whereby the user can easily recall the "amount of exercise" simply by glancing at the display of the icons. The icons from which to recall the amount of exercise are not limited to the icons imitating the sweats.

Further, the amount of exercise may be set selectively from three types such as 100% given when walking, e.g., 10,000 or more steps, 80% given when walking 8,000 steps or more but less than 10,000 steps and 50% given when walking 8,000 steps or less. The blood sugar level measuring apparatus 1 may incorporate a pedometer which counts the number of steps of the user, and the user may also carry the pedometer. In the process of S401 of the measurement history display process explained in the first working example, the user conducts the slide operation of the operation button 7, thereby displaying the icons imitating the sweats on the liquid crystal screen 4. Concretely, the icon Ic1 for the post-exercise flag is displayed in the icon display region DR3, and the icons imitating the sweats are displayed together with the identification number and the measurement date/time of the measured result in the graph display region DR1.

The number of steps of the user is inputted as the amount of exercise in S502, in which case the number of steps of the user may be determined as the amount of exercise corresponding to the post-exercise flag. Then, the number of steps of the user determined as the amount of exercise is recorded on the recording device in the blood sugar level measuring apparatus 1. Moreover, the number of steps of the user is converted into a quantity of energy consumption, and this quantity of energy consumption may be determined as the amount of exercise corresponding to the post-exercise flag. Then, the quantity of energy consumption determined as the amount of exercise is recorded on the recording device in the blood sugar level measuring apparatus 1. If the blood sugar level measuring apparatus 1 incorporates the pedometer, at the point of time when the post-exercise flag is selected in S502, the number of steps of the user and the quantity of energy consumption may also be recorded on the recording device in the blood sugar level measuring apparatus 1. Note that the user performs the slide operation of the operation button 7 in the process of S401 of the measurement history display process explained in the first working example, whereby the number of steps of the user and the quantity of energy consumption recorded on the recording device in the blood sugar level measuring apparatus 1 are displayed on the liquid crystal screen 4. Specifically, the number of steps of the user and the quantity of energy consumption are displayed together with the identification number and the measurement date/time of the measured result in the graph display region DR1. Upon finishing S512, the processing advances to S513.

It is determined in S513 whether the elapse time counter is set in the blood sugar level measuring apparatus 1 or not. The elapse time counter is a function set by the elapse time setting unit 32 of the blood sugar level measuring apparatus 1 in advance of the measurement and is also a function of automatically counting the elapse time since the point of time when starting the exercise and associating the elapse time with the blood sugar level given as the measured result. Note that this elapse time is displayed as post-exercise elapse time Ft in the icon display region DR3 in FIG. 15. The user is thereby enabled to recognize, in addition to the amount of exercise, how much time has elapsed since the start of the exercise when the blood sugar level is measured, and hence the health management about the blood sugar level is facilitated. Further, this elapse time may also be displayed as the post-exercise elapse time Ft in the graph display region DR1. The processing advances to S514 if determined to be affirmative in S513 but diverts to S515 whereas if determined to be negative.

The setting is done in S514 so that the icon corresponding to the post-exercise flag and the post-exercise elapse time Ft are simultaneously displayed on the liquid crystal screen 4. The setting is made so that the icon Ic1 for the post-exercise flag and the post-exercise elapse time Ft are displayed in the icon display region DR3, while the icons imitating the sweats are displayed in the graph display region DR1. The number of icons imitating the sweats is set corresponding to the amount of exercise. Furthermore, the setting may be done so as to display the post-exercise elapse time Ft in the graph display region DR1. When S514 comes to an end, the processing advances to S521. While on the other hand, the setting is made in S515 so that the post-exercise elapse time Ft is not displayed on the liquid crystal screen 4, the icon Ic1 for the post-exercise flag is displayed in the icon display region DR3, and the icons imitating the sweats are displayed in the graph display region DR1. The number of icons imitating the sweats is set corresponding to the amount of exercise. Upon finishing S515, the processing advances to S521.

Described next is a case in which the negative determination is made in S511A and the processing advances to S516. It is determined in S516 whether the post-insulin-dosing flag is selected or not. Herein, the processing advances to S517 if determined to be affirmative but diverts to S521 whereas if determined to be negative.

In S517, the amount of dosage of insulin, which corresponds to the post-insulin-dosing flag, is determined. Owing to this process, the accompanying data on how much amount of dosage of insulin the user has taken when making the measurement can be associated with the post-insulin-dosing flag set by the blood sugar level measuring apparatus 1. For example, the amount of dosage of insulin may be set selectively from three types such as "the amount of dosage of insulin being large" if the amount of dosage of insulin is larger than a predetermined quantity, "the amount of dosage of insulin being the same" if the amount of dosage of insulin is substantially the same as the predetermined quantity, and "the amount of dosage of insulin being small" if the amount of dosage of insulin is smaller than the predetermined quantity. The predetermined quantity is a value that can be arbitrarily set by the user.

The post-insulin-dosing flag is, as depicted in FIG. 16, selected in a status where an icon Ic5 related to the post-insulin-dosing flag is displayed in the graph display region DR1. Then, the amount of dosage of insulin is, as illustrated in FIG. 16, selected in a status where an icon Ic6 related to the amount of dosage of insulin is displayed in the graph display region DR1 in a way that changes the number of bars displayed within the icon. For instance, the case of "the amount of dosage of insulin being large" is indicated by three lengths of bars (the status depicted in FIG. 16), and the cases of being "the same" and "small" are indicated by two bars and one bar, respectively. Thus, the data on the amount of dosage of insulin is associated with the post-insulin-dosing flag, thereby enabling the user to know how much amount of dosage of insulin the user has taken when the blood sugar level is measured, to understand the relation between the amount of dosage of insulin and the blood sugar level, and facilitating the health management in regard to the blood sugar level. Note that the icon Ic5 related to the post-insulin-dosing flag takes a shape imitating a syringe, and the icon Ic6 related to the amount of dosage of insulin takes a shape imitating a medicine bottle. Then, one through three bars are displayed within the medicine bottle, thereby enabling the user to easily recall the "amount of dosage of insulin" simply by glancing at the icons displayed therein. Further, the icon Ic5 related to the post-insulin-dosing flag is not limited to the shape imitating the syringe. Moreover, the icon Ic6 related to the amount of dosage of insulin is not limited to the shape imitating the medicine bottle.

The user conducts the slide operation of the operation button 7 in the process of S401 of the measurement history display process explained in the first working example, whereby the icon Ic5 related to the post-insulin-dosing flag and the icon Ic6 related to the amount of dosage of insulin are displayed on the liquid crystal screen 4. Concretely, the icon Ic5 related to the post-insulin-dosing flag and the icon Ic6 related to the amount of dosage of insulin are displayed together with the identification number and the measurement date/time of the measured result in the graph display region DR1.

If the amount of dosage (unit) of insulin is inputted in S502, the amount of dosage (unit) of insulin is recorded on the recording device in the blood sugar level measuring apparatus 1. Note that the user carries out the slide operation of the operation button 7 in the process of S401 of the measurement history display process described in the first working example, thereby displaying the amount of dosage (unit) of insulin, which is recorded on the recording device in the blood sugar level measuring apparatus 1, on the liquid crystal screen 4. To be specific, the amount of dosage (unit) of insulin is displayed together with the identification number and the measurement date/time of the measured result in the graph display region DR1. When finishing S517, the processing advances to S518.

It is determined in S518 whether the elapse time counter is set in the blood sugar level measuring apparatus 1 or not. The elapse time counter is the function set by the elapse time setting unit 32 of the blood sugar level measuring apparatus 1 in advance of the measurement and is also the function of automatically counting the elapse time since the point of time when dosing the insulin and associating the elapse time with the blood sugar level given as the measured result. Note that this elapse time is displayed as post-insulin-dosing elapse time Gt in the icon display region DR3 in FIG. 16. The user is thereby enabled to recognize, in addition to the amount of dosage of insulin, how much time has elapsed since after dosing the insulin when the blood sugar level is measured, and hence the health management about the blood sugar level is facilitated. Further, this elapse time may also be displayed as the post-insulin-dosing elapse time Gt in the graph display region DR1. The processing advances to S519 if determined to be affirmative in S518 but diverts to S520 whereas if determined to be negative.

The setting is done in S519 so that the icon corresponding to the post-insulin-dosing flag and the post-insulin-dosing elapse time Gt are simultaneously displayed on the liquid crystal screen 4. The setting is made so that the post-insulin-dosing elapse time Gt is displayed in the icon display region DR3, while the icon Ic5 related to the post-insulin-dosing flag and the icon Ic6 related to the amount of dosage of insulin are displayed in the graph display region DR1. The number of bars within the icon Ic6 related to the amount of dosage of insulin is set corresponding to the amount of dosage of insulin. Upon an end of S519, the processing advances to S521. While on the other hand, the setting is done in S520 so that the post-insulin-dosing elapse time Gt is displayed neither in the graph display region DR1 nor in the icon display region DR3, while the icon Ic5 related to the post-insulin-dosing flag and the icon Ic6 related to the amount of dosage of insulin are displayed in the graph display region DR1. The number of bars within the icon Ic6 related to the amount of dosage of insulin is set corresponding to the amount of dosage of insulin. Upon an end of S520, the processing advances to S521.

Processes from S521 (FIG. 14) onward will be described. It is determined in S521 whether the β-cell flag is selected in S502 or not. Herein, the processing advances to S522 if determined to be affirmative but diverts to S524 whereas if determined to be negative.

In S522, an amount of β cells corresponding to the β-cell flag is determined. Owing to this process, the accompanying data on the amount of β cells of the user when making the measurement can be associated with the β-cell flag set by the blood sugar level measuring apparatus 1. For instance, the amount of β cells may be set selectively from three types such as "the amount of β cells being large" if the amount of β cells is larger than a predetermined quantity, "the amount of β cells being the same" if the amount of β cells is substantially the same as the predetermined quantity, and "the amount of β cells being small" if the amount of β cells is smaller than the predetermined quantity. The predetermined quantity is a value that can be arbitrarily set by the user. The amount of β cells may also be measured by the known technology.

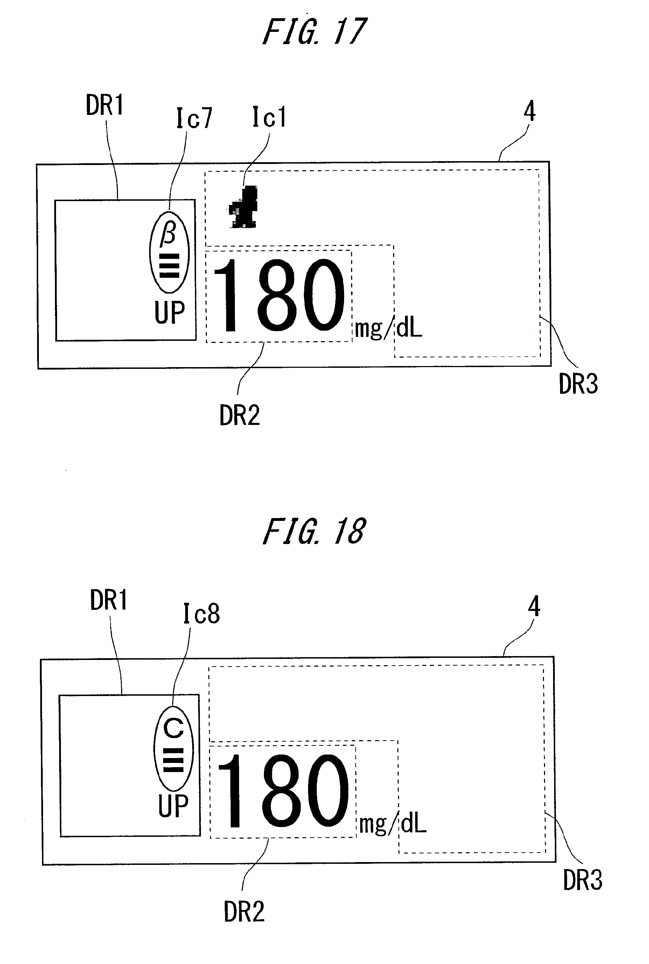

The selection of the β-cell flag and the selection of the amount of β cells are, as illustrated in FIG. 17, conducted in a status where an icon Ic7 about the β-cell flag is displayed in the graph display region DR1 in a way that changes the number of bars displayed within the icon. For instance, the case of "the amount of β cells being large" is indicated by three lengths of bars (the status depicted in FIG. 17), and the cases of being "the same" and "small" are indicated by two bars and one bar, respectively. Thus, the data on the amount of β cells is associated with the β-cell flag, thereby enabling the user to know the amount of β cells when making the measurement, to understand the relation between the amount of β cells and the blood sugar level, and facilitating the health management in regard to the blood sugar level.

The user performs the slide operation of the operation button 7 in the process of S401 of the measurement history display process described in the first working example, whereby the icon Ic7 related to the β-cell flag is displayed on the liquid crystal screen 4. Specifically, the icon Ic7 related to the β-cell flag is displayed together with the identification number and the measurement date/time of the measured result in the graph display region DR1.

If the amount of β cells is inputted in S502, the amount of β cells is recorded on the recording device in the blood sugar level measuring apparatus 1. Note that the user carries out the slide operation of the operation button 7 in the process of S401 of the measurement history display process described in the first working example, thereby displaying the amount of β cells, which is recorded on the recording device in the blood sugar level measuring apparatus 1, on the liquid crystal screen 4. To be specific, the amount of β cells is displayed together with the identification number and the measurement date/time of the measured result in the graph display region DR1. When finishing S522, the processing advances to S523.

In S523, a change in amount of β cells corresponding to the β-cell flag is determined. Through this process, the accompanying data on the change in amount of β cells of the user when making the measurement can be associated with the β-cell flag set by the blood sugar level measuring apparatus 1. For example, a change in amount of β cells may be set selectively from three types such as "the amount of β cells being UP" if the amount of β cells in measuring the blood sugar level this time is larger than the amount of βcells in measuring the blood sugar level last time, "the amount of β cells being the SAME" if the amount of β cells (of this time) is substantially the same as the amount of β cells (of the last time) and "the amount of β cells being DOWN" if the amount of β cells (of this time) is smaller than the amount of β cells (of the last time).

The change in amount of β cells is, as illustrated in FIG. 17, selected in a status where the icon Ic7 related to the β-cell flag is displayed in the graph display region DR1 in a way that changes a character "UP" (the status depicted in FIG. 17), a character "SAME" and a character "DOWN" displayed in the graph display region DR1. The character "UP", the character "SAME" and the character "DOWN" displayed in the graph display region DR1 are pieces of information each representing the change in amount of β cells. Thus, the data on the change in amount of β cells is associated with the β-cell flag, thereby enabling the user to know the change in amount of β cells when making the measurement, to understand the relation between the change in amount of β cells and the blood sugar level, and facilitating the health management in regard to the blood sugar level. Note that a history of the amount of β cells may be recorded on the recording device in the blood sugar level measuring apparatus 1. Through this process, when the β-cell flag and the amount of β cells are selected, the amount of β cells (of this time) is compared with the amount of β cells (of the last time), thus determining the change in amount of β cells.

The user performs the slide operation of the operation button 7 in the process of S401 of the measurement history display process described in the first working example, thereby displaying the icon Ic7 related to the β-cell flag and the information representing the change in amount of β cells on the liquid crystal screen 4. To be specific, the icon Ic7 related to the β-cell flag and the information representing the change in amount of β cells are displayed together with the identification number and the measurement date/time of the measured result in the graph display region DR1.

If the amount of β cells is inputted in S502, the amount of β cells is recorded on the recording device in the blood sugar level measuring apparatus 1. Note that the user carries out the slide operation of the operation button 7 in the process of S401 of the measurement history display process described in the first working example, thereby displaying the amount of β cells, which is recorded on the recording device in the blood sugar level measuring apparatus 1, on the liquid crystal screen 4. Concretely, the amount of β cells is displayed together with the identification number and the measurement date/time of the measured result in the graph display region DR1. When finishing S523, the processing advances to S524.

It is determined in S524 whether the cholesterol flag is selected in S502 or not. Herein, the processing advances to S525 if determined to be affirmative but diverts to S527 whereas if determined to be negative.

In S525, a cholesterol value corresponding to the cholesterol flag is determined. Owing to this process, the accompanying data on the cholesterol value of the user when making the measurement can be associated with the cholesterol flag set by the blood sugar level measuring apparatus 1. The cholesterol value may be any one of a total cholesterol (T-chol) value, an HDL cholesterol (HDL-chol) value and an LDL cholesterol (LDL-chol) value. For instance, the cholesterol value may set selectively from three types such as "the cholesterol value of the user being higher than a predetermined value", "the cholesterol value of the user being substantially the same as the predetermined value" and "the cholesterol value of the user being lower than the predetermined value". The predetermined value is a value that can be arbitrarily set by the user. The cholesterol value may also be measured by the known technology.

The selection of the cholesterol flag and the selection of the cholesterol value are, as illustrated in FIG. 18, conducted in a status where an icon Ic8 about the cholesterol flag is displayed in the graph display region DR1 in a way that changes the number of bars displayed within the icon. For example, the case of "the cholesterol value being high" is indicated by three lengths of bars (the status depicted in FIG. 18), and the cases of being "substantially the same" and "lower" are indicated by two bars and one bar, respectively. Thus, the data on the cholesterol value is associated with the cholesterol flag, thereby enabling the user to know the cholesterol value when making the measurement, to understand the relation between the cholesterol value and the blood sugar level, and facilitating the health management in regard to the blood sugar level.

The user performs the slide operation of the operation button 7 in the process of S401 of the measurement history display process described in the first working example, thereby displaying the icon Ic8 related to the cholesterol flag on the liquid crystal screen 4. Concretely, the icon Ic8 related to the cholesterol flag is displayed together with the identification number and the measurement date/time of the measured result in the graph display region DR1.

If the cholesterol value is inputted in S502, the cholesterol value is recorded on the recording device in the blood sugar level measuring apparatus 1. Note that the user carries out the slide operation of the operation button 7 in the process of S401 of the measurement history display process described in the first working example, thereby displaying the cholesterol value, which is recorded on the recording device in the blood sugar level measuring apparatus 1, on the liquid crystal screen 4. To be specific, the cholesterol value is displayed together with the identification number and the measurement date/time of the measured result in the graph display region DR1. Upon finishing S525, the processing advances to S526.

In S526, a change in cholesterol value corresponding to the cholesterol flag is determined. Owing to this process, the accompanying data on the change in cholesterol value of the user when making the measurement can be associated with the cholesterol flag set by the blood sugar level measuring apparatus 1. For instance, the change in cholesterol value may be set selectively from three types such as "the cholesterol value being UP" if the cholesterol value in measuring the blood sugar level this time is larger than the cholesterol value in measuring the blood sugar level last time, "the cholesterol value being the SAME" if the cholesterol value (of this time) is substantially the same as the cholesterol value (of the last time) and "the cholesterol value being DOWN" if the cholesterol value (of this time) is smaller than the cholesterol value (of the last time).

The change in cholesterol value is, as illustrated in FIG. 18, selected in a status where the icon Ic8 related to the cholesterol flag is displayed in the graph display region DR1 in a way that changes the character "UP" (the status depicted in FIG. 18), the character "SAME" and the character "DOWN" displayed in the graph display region DR1. The character "UP", the character "SAME" and the character "DOWN" displayed in the graph display region DR1 are pieces of information each representing the change in cholesterol value. Thus, the data on the change in cholesterol value is associated with the cholesterol flag, thereby enabling the user to know the change in cholesterol value when making the measurement, to understand the relation between the change in cholesterol value and the blood sugar level, and facilitating the health management in regard to the blood sugar level. Note that a history of the cholesterol value may be recorded on the recording device in the blood sugar level measuring apparatus 1. Through this process, when the cholesterol flag and the cholesterol value are selected, the cholesterol value (of this time) is compared with the cholesterol value (of the last time), thus determining the change in cholesterol value.

The user performs the slide operation of the operation button 7 in the process of S401 of the measurement history display process described in the first working example, thereby displaying the icon Ic8 related to the cholesterol flag and the information representing the change in cholesterol value on the liquid crystal screen 4. Concretely, the icon Ic8 related to the cholesterol flag and the information representing the change in cholesterol value are displayed together with the identification number and the measurement date/time of the measured result in the graph display region DR1.

If the cholesterol value is inputted in S502, the cholesterol value is recorded on the recording device in the blood sugar level measuring apparatus 1. Note that the user carries out the slide operation of the operation button 7 in the process of S401 of the measurement history display process described in the first working example, thereby displaying the cholesterol value, which is recorded on the recording device in the blood sugar level measuring apparatus 1, on the liquid crystal screen 4. To be specific, the cholesterol value is displayed together with the identification number and the measurement date/time of the measured result in the graph display region DR1. Upon finishing S526, the processing advances to S527.

It is determined in S527 whether the cholesterol flag is selected in S502 or not. Herein, if determined to be affirmative, the processing advances to S528, and, whereas if determined to be negative, the measured result management process is terminated.

In S528, the HbA1C value corresponding to the HbA1C flag is determined. Owing to this process, the accompanying data on the HbA1C value of the user when making the measurement can be associated with the HbA1C flag set by the blood sugar level measuring apparatus 1. For instance, the HbA1C value may be set selectively from three types such as "the user's HbA1C value being higher than a predetermined value", "the user's HbA1C value being substantially the same as the predetermined value" and "the user' s HbA1C value being lower than the predetermined value". The predetermined value is a value that can be arbitrarily set by the user. The HbA1C value may also be measured by the known technology.

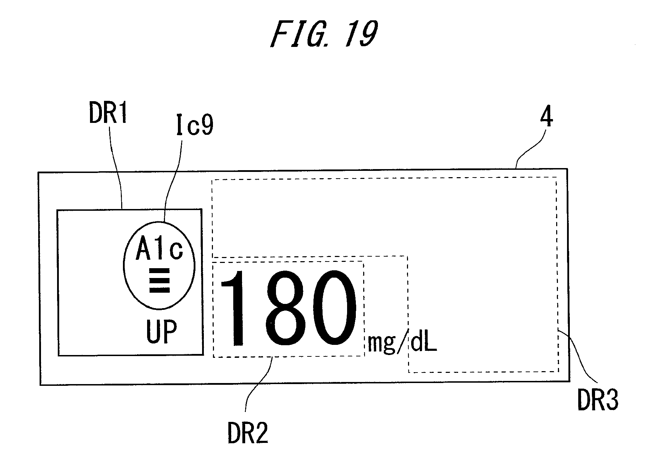

The selection of the HbA1C flag and the selection of the HbA1C value are, as illustrated in FIG. 19, conducted in a status where an icon Ic9 about the HbA1C flag is displayed in the graph display region DR1 in a way that changes the number of bars displayed within the icon. For example, the case of "the HbA1C value being high" is indicated by three lengths of bars (the status depicted in FIG. 19), and the cases of being "substantially the same" and "lower" are indicated by two bars and one bar, respectively. Thus, the data on the HbA1C value is associated with the HbA1C flag, thereby enabling the user to know the HbA1C value when making the measurement, to understand the relation between the HbA1C value and the blood sugar level, and facilitating the health management in regard to the blood sugar level.

The user performs the slide operation of the operation button 7 in the process of S401 of the measurement history display process described in the first working example, thereby displaying the icon Ic9 related to the HbA1C flag on the liquid crystal screen 4. Specifically, the icon Ic9 related to the HbA1C flag is displayed together with the identification number and the measurement date/time of the measured result in the graph display region DR1.

If the HbA1C value is inputted in S502, the HbA1C value is recorded on the recording device in the blood sugar level measuring apparatus 1. Note that the user carries out the slide operation of the operation button 7 in the process of S401 of the measurement history display process described in the first working example, thereby displaying the HbA1C value, which is recorded on the recording device in the blood sugar level measuring apparatus 1, on the liquid crystal screen 4. To be specific, the HbA1C value is displayed together with the identification number and the measurement date/time of the measured result in the graph display region DR1. Upon finishing S528, the processing advances to S529.

In S529, a change in HbA1C value corresponding to the HbA1C flag is determined. Owing to this process, the accompanying data on the change in HbA1C value of the user when making the measurement can be associated with the HbA1C flag set by the blood sugar level measuring apparatus 1. For instance, the change in HbA1C value may be set selectively from three types such as "the HbA1C value being UP" if the HbA1C value in measuring the blood sugar level this time is larger than the HbA1C value in measuring the blood sugar level last time, "the HbA1C value being the SAME" if the HbA1C value (of this time) is substantially the same as the HbA1C value (of the last time) and "the HbA1C value being DOWN" if the HbA1C value (of this time) is smaller than the HbA1C value (of the last time).

The change in HbA1C value is, as illustrated in FIG. 19, selected in a status where the icon Ic9 related to the HbA1C flag is displayed in the graph display region DR1 in a way that changes the character "UP" (the status depicted in FIG. 19), the character "SAME" and the character "DOWN" displayed in the graph display region DR1. The character "UP", the character "SAME" and the character "DOWN" displayed in the graph display region DR1 are pieces of information each representing the change in HbA1C value. Thus, the data on the change in HbA1C value is associated with the HbA1C flag, thereby enabling the user to know the change in HbA1C value when making the measurement, to understand the relation between the change in HbA1C value and the blood sugar level, and facilitating the health management in regard to the blood sugar level. Note that a history of the HbA1C value may be recorded on the recording device in the blood sugar level measuring apparatus 1. Through this process, when the HbA1C flag and the HbA1C value are selected, the HbA1C value (of this time) is compared with the HbA1C value (of the last time), thus determining the change in HbA1C value.

The user performs the slide operation of the operation button 7 in the process of S401 of the measurement history display process described in the first working example, thereby displaying the icon Ic9 related to the HbA1C flag and the information representing the change in HbA1C value on the liquid crystal screen 4. Concretely, the icon Ic9 related to the HbA1C flag and the information representing the change in HbA1C value are displayed together with the identification number and the measurement date/time of the measured result in the graph display region DR1.

If the HbA1C value is inputted in S502, the HbA1C value is recorded on the recording device in the blood sugar level measuring apparatus 1. Note that the user carries out the slide operation of the operation button 7 in the process of S401 of the measurement history display process described in the first working example, thereby displaying the HbA1C value, which is recorded on the recording device in the blood sugar level measuring apparatus 1, on the liquid crystal screen 4. To be specific, the HbA1C value is displayed together with the identification number and the measurement date/time of the measured result in the graph display region DR1. Upon finishing S529, the measured result management process is terminated.

The graph display region DR1 of the liquid crystal screen 4 is configured to include dot display elements arrayed in matrix, and therefore the icons and the marks displayed in the graph display region DR1 of the liquid crystal screen 4 can be changed without any restriction. Accordingly, the icons and the marks displayed in the graph display region DR1 of the liquid crystal screen 4 are not limited to the shapes and patterns of the icons and marks explained in the third working example but can adopt a variety of shapes and patterns. It is to be noted that various items of information can be, without being limited to the icons and marks, displayed in the graph display region DR1 of the liquid crystal screen 4. Further, in the third working example, the whole of the graph display regions DR1, the DR2 and the DR3 of the liquid crystal screen 4 may be configured to include the dot display elements arrayed in matrix. With this configuration, it is feasible to change, without any restriction, the icons and the marks displayed in the graph display region DR1, the DR2 and the DR3 of the liquid crystal screen 4. Accordingly, the icons and marks displayed in the graph display regions DR1, the DR2 and the DR3 of the liquid crystal screen 4 can adopt the variety of shapes and patterns without being limited to the shapes and the patterns of the icons and the marks described in the third working example. Further, the various items of information can be, without being limited to the icons and the marks, displayed in the graph display region DR1 of the liquid crystal screen 4. For example, the information displayed on the liquid crystal screen 4 may also be displayed as a dynamic or moving image such as animation.

### <System Architecture>

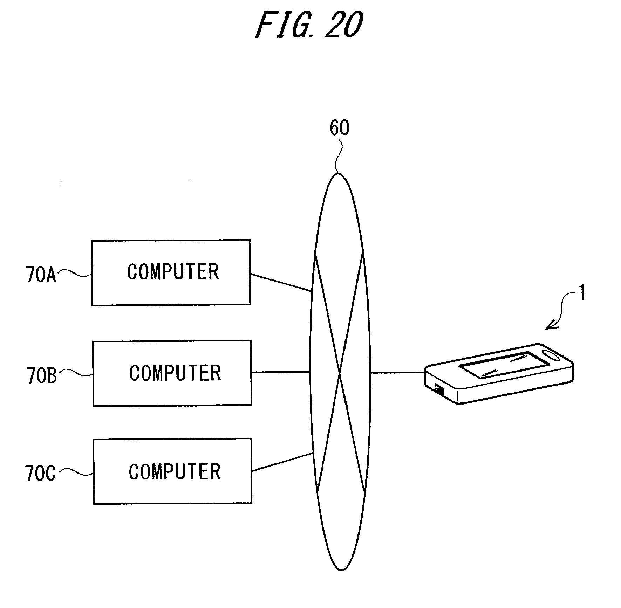

FIG. 20 is a view of a system architecture of the present information management system. The information management system is configured to include the blood sugar level measuring apparatus 1, a network 60, and computers 70A, 70B, 70C, etc that are connected via the network 60 to the blood sugar level measuring apparatus 1. The network 60 is exemplified by communication liens such as the Internet, an intranet, a telephone line, a private line, an optical communication network and a communication satellite. The computers 70A, 70B, 70C are external apparatuses each including a CPU, a ROM, a RAM, an external storage device, a communication interface, etc. The computers 70A, 70B, 70C are generically referred to as the computers 70. FIG. 20 illustrates the three computers 70A, 70B, 70C, however, the number of the computers 70 is not limited to "3", and one single computer 70 or a plurality of computers 70 may also be available. The blood sugar level measuring apparatus 1 and the computers 70 are connected to each other via the network 60 by communication port 8 of the blood sugar level measuring apparatus 1. The management control unit 30 of the blood sugar level measuring apparatus 1 accesses the computer 70 via the external communication unit 50 and accepts an access from the computer 70. Specifically, the management control unit 30 of the blood sugar level measuring apparatus 1 transmits the information recorded on the recording device in the blood sugar level measuring apparatus 1 to the computer 70 via the external communication unit 50, and receives the information managed by the computer 70. The computers 70 are installed in medical institutions such as hospitals and may also be, without being limited to within these institutions, installed as so-called external servers outside the medical institutions like the hospitals. Further, the computers 70A, 70B, 70C may also be installed respectively in each hospital. Moreover, the computer 70 may also be used as a management server which integrates and thus manages the items of information managed by a plurality of hospitals in an integrated manner.

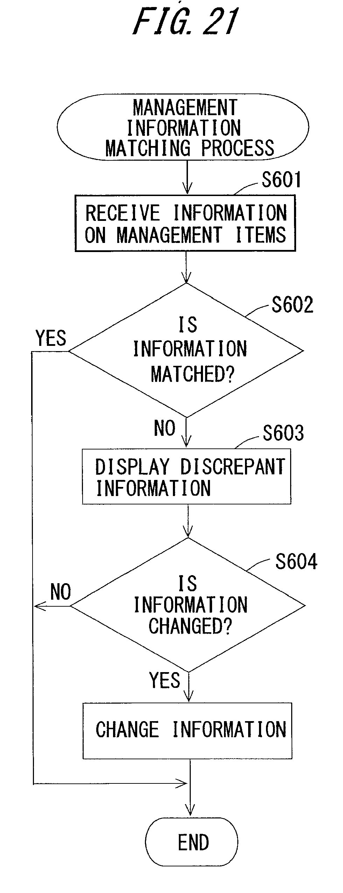

Next, the management information matching process executed by the management control unit 30 will be described based on FIG. 21. The management information matching process is a process to match the information managed by the blood sugar level measuring apparatus 1 with at least a part of the information managed by the computer 70. The blood sugar level measuring apparatus 1 receives the information managed by the respective computers from the computers 70A, 70B, 70C, in which case the management control unit 30 selects any one of these computers and may execute the management information matching process. At first, in S601, the management control unit 30 receives the information on the items managed by the computer 70 via the external communication unit 50. That is, the management control unit 30 accepts the input of the information managed by the computer 70 via the external communication unit 50. When finishing the process in S601, the processing advances to S602.

It is determined in S602 whether or not the information managed by the blood sugar level measuring apparatus 1 matches with the information managed by the computer 70. The information managed by the blood sugar level measuring apparatus 1 and the information managed by the computer 70 are every category of biometric information managed with respect to the blood sugar level such as the amount of β-cells, the cholesterol value and the HbA1C value. The determination how much the information managed by the blood sugar level measuring apparatus 1 is matched with the information managed by the computer 70 may be made based on partial matching or complete matching. Further, even if the information managed by the blood sugar level measuring apparatus 1 is not completely matched with the information managed by the computer 70 but if the information managed by the computer 70 falls within an allowable range, it may be determined that these pieces of information are matched with each other. If determined to be affirmative in S602, such a message is displayed on the liquid crystal screen 4 as to purport that the information managed by the blood sugar level measuring apparatus 1 is matched with the information managed by the computer 70, and the management information matching process is finished. Whereas if determined to be negative in S602, the processing advances to S603.

In S603, such information is displayed on the liquid crystal screen 4 as to indicate discrepancy between the information managed by the blood sugar level measuring apparatus 1 and the information managed by the computer 70. For example, if there is discrepancy, such that some items managed by the computer 70 are not managed by the blood sugar level measuring apparatus 1, between the information managed by the blood sugar level measuring apparatus 1 and the information managed by the computer 70, such a message is displayed on the liquid crystal screen 4 as to purport that the management items of the blood sugar level measuring apparatus 1 are deficient (or excessive). The management items are individual pieces of information managed by the blood sugar level measuring apparatus 1 and include, e.g., the pieces of data themselves such as the blood sugar level and the amount of β cells managed by the blood sugar level measuring apparatus 1, and data names as well. Upon finishing the process in S603, the processing advances to S604.

It is determined in S604 whether or not the management items of the blood sugar level measuring apparatus 1 are changed (added or deleted) with respect to discrepant pieces of information between the information managed by the blood sugar level measuring apparatus 1 and the information managed by the computer 70. For instance, the information managed by the blood sugar level measuring apparatus 1 and the information managed by the computer 70 are listed, and such a message is displayed on the liquid crystal screen 4 as to indicate whether the management items of the blood sugar level measuring apparatus 1 are changed or not. The user determines by operating the operation button 7 whether a process of changing the management items is established or not. The user can select the information that should be changed (added or deleted) and the information that should not be changed (added or deleted) with respect to the plurality of discrepant management items between the information managed by the computer 70 and the information managed by the blood sugar level measuring apparatus 1. For example, when the amount of β cells and the cholesterol value other than the blood sugar level are displayed as the information managed by the computer 70 on the liquid crystal screen 4, the amount of β cells may be added to the memory, but the cholesterol value may not be added to the memory. If determined to be affirmative in S604, the processing advances to S605. Whereas if determined to be negative in S604, the management information matching process comes to an end.

With respect to the management items determined or selected in S604, the information managed by the blood sugar level measuring apparatus 1 is changed (added or deleted) in S605. Concretely, the management items determined or selected in S604 are added to the memory of the blood sugar level measuring apparatus 1, and the management items determined or selected in S604 are deleted from the memory of the blood sugar level measuring apparatus 1. With this process, the information managed by the blood sugar level measuring apparatus 1 can be matched with at least a part of the information managed by the computer 70. The management items of the blood sugar level measuring apparatus 1 can be thereby properly changed in a way that adds the items desired to be managed especially also by the user according to the request in the items managed by the computer 70 installed at, e.g., the hospital. Note that if the information on the flag is newly changed in the memory of the blood sugar level measuring apparatus 1, the management control unit 30 properly reads the predetermined flag information from the memory into a predetermined location, and this information is displayed in the graph display region DR1.

The management information matching process executed by the management control unit 30 may be carried out in such a way that the user, e.g., inputs the information directly to the memory of the blood sugar level measuring apparatus 1. For instance, the user operates the operation button 7 and a separately provided operation means, whereby the information managed by the computer 70 and the information on the blood sugar level may also be inputted to the blood sugar level measuring apparatus 1. As described above, after the information on the flag has been newly changed in the memory of the blood sugar level measuring apparatus 1, the management control unit 30 properly reads the predetermined flag information from the memory into the predetermined location, and the readout information is displayed in the graph display region DR1.

It is to be noted that the dot display unit capable of displaying the icons without the restrictions is limited to the graph display region DR1 in the working examples discussed above, however, the liquid crystal screen 4 is configured to enable the dot display to be attained on the whole and may also be configured to enable the desired flag to be displayed properly. Note that the blood sugar level measuring apparatus 1 can, in the case of taking the configuration capable of changing the flag as described above, record the measurement data corresponding to the flag to be changed (added/deleted) on the recording unit.

Note that the blood sugar level measuring apparatus 1 can be configured to match at least a part of the management information with information in another blood sugar level measuring apparatus by the same mechanism as described above.

### DESCRIPTION OF THE REFERENCE NUMERALS AND SYMBOLS

1 blood sugar level measuring apparatus
2 elongate housing
4 liquid crystal screen
7 operation button
20 measurement control unit
30 management control unit
40 display control unit
50 external communication unit
60 network
70A, 70B, 70C computer

## Claims

1. In a blood sugar level measuring apparatus comprising:
a measuring unit to measure a blood sugar level of a user;
a recording unit to record measurement data thereof; and
a display unit to capable of displaying a measured result on a display,
the improvement **characterized by** further comprising:
a condition flag setting unit to record, on the recording unit, a condition flag related to a body condition of the user when measuring the blood sugar level in a way that associates the condition flag with blood sugar level data measured by the measuring unit; and
a display control unit to display the measured result recorded on the recording unit on the display on the basis of the condition flag set by the condition flag setting unit.

2. The blood sugar level measuring apparatus according to claim 1, further comprising a matching unit to match the information managed by the blood sugar level measuring apparatus with at least a part of information managed by an external apparatus.

3. The blood sugar level measuring apparatus according to claim 1 or 2, wherein the display control unit displays graphs of transitions of the blood sugar levels of the user on the display on the basis of the measurement data recorded on the recording unit.

4. The blood sugar level measuring apparatus according to claim 3, wherein the display control unit displays predetermined marks corresponding to the respective condition flags associated with plural pieces of blood sugar level data indicated by the graphs in the way of being adjacent to the graphs.

5. The blood sugar level measuring apparatus according to claim 1, wherein the display control unit displays an arithmetic value based on the measurement data recorded on the recording unit on the display.

6. The blood sugar level measuring apparatus according to claim 5, wherein the display control unit extracts a measurement data group exhibiting the same condition flag set by the condition flag setting unit from within the measurement data recorded on the recording unit, and displays the arithmetic value about the extracted measurement data group on the display.

7. The blood sugar level measuring apparatus according to claim 5 or 6, wherein the arithmetic value is an average value of the blood sugar levels of the user for a predetermined period.

8. The blood sugar level measuring apparatus according to any one of claims 1 through 7, wherein the condition flag associated by the condition flag setting unit is a flag about at least one of a pre-meal condition, a post-meal condition, a post-exercise condition, a post-medicine-dosing condition and an in-vivo substance quantity condition of the user.

9. The blood sugar level measuring apparatus according to claim 8, wherein when the condition flag associated by the condition flag setting unit is the flag about the post-meal condition, the display control unit displays information on an amount of meal of the user together with the blood sugar level of a blood of the user, which is measured by the measuring unit, on the display.

10. The blood sugar level measuring apparatus according to claim 9, wherein the information on the amount of meal of the user is displayed as an image imitating the stomach of a person and as a variation image that varies corresponding to the amount of meal.

11. The blood sugar level measuring apparatus according to any one of claims 8 through 10, wherein when the condition flag associated by the condition flag setting unit is the flag about the post-meal flag, the display control unit displays, on the display, information on elapse time since at least one of a point of time when the user starts having the meal, a predetermined point of time during the meal and a point of time when completing the meal together with the blood sugar level of the blood of the user that is measured by the measuring unit.

12. The blood sugar level measuring apparatus according to any one of claims 8 through 11, further comprising an operation unit through which the user can give an instruction to associate the condition flag with the blood sugar level data,
wherein the condition flag setting unit further associates the information on the amount of meal with the blood sugar level data associated with the flag about the post-meal condition on the basis of a user's operation over the operation unit.

13. The blood sugar level measuring apparatus according to claim 12, wherein the user's operation over the operation unit is an operation of inputting or selecting a predetermined condition about a content of the meal, and
the condition flag setting unit automatically calculates the information on the amount of meal according to a result of the operation, and associates this information with the blood sugar level data.

14. A blood sugar level measured result display method of displaying, in a blood sugar level measuring apparatus comprising: a measuring unit to measure a blood sugar level of a user; a recording unit to record measurement data thereof; and a display unit to capable of displaying a measured result on a display, the measured result on the display, the method comprising:
a condition flag setting step of recording, on the recording unit, a condition flag related to a body condition of the user when measuring the blood sugar level in a way that associates the condition flag with measured blood sugar level data; and
a display control step of displaying the measured result recorded on the recording unit on the display on the basis of the condition flag set in the condition flag setting step.

15. The blood sugar level measured result display method according to claim 14, further comprising a matching step of matching the information managed by the blood sugar level measuring apparatus with at least a part of information managed by an external apparatus.

16. The blood sugar level measured result display method according to claim 14 or 15, wherein the display control step includes displaying graphs of transitions of the blood sugar levels of the user on the display on the basis of the measurement data recorded on the recording unit.

17. The blood sugar level measured result display method according to claim 16, wherein the display control step includes displaying predetermined marks corresponding to the respective condition flags associated with plural pieces of blood sugar level data indicated by the graphs in the way of being adjacent to the graphs.

18. The blood sugar level measured result display method according to claim 14, wherein the display control step includes displaying an arithmetic value based on the measurement data recorded on the recording unit on the display.

19. The blood sugar level measured result display method according to claim 18, wherein the display control step includes extracting a measurement data group exhibiting the same condition flag set by the condition flag setting unit from within the measurement data recorded on the recording unit, and displaying the arithmetic value about the extracted measurement data group on the display.

20. The blood sugar level measured result display method according to claim 18 or 19, wherein the arithmetic value is an average value of the blood sugar levels of the user for a predetermined period.

21. A blood sugar level measured result display control program as a computer program to make a computer comprising:
a measuring unit to measure a blood sugar level of a user;
a recording unit to record measurement data thereof; and a display unit to capable of displaying a measured result on a display, display the measured result on the display, the program making the computer execute:
a condition flag setting step of recording, on the recording unit, a condition flag related to a body condition of the user when measuring the blood sugar level in a way that associates the condition flag with measured blood sugar level data; and
a display control step of displaying the measured result recorded on the recording unit on the display on the basis of the condition flag set in the condition flag setting step.

22. The blood sugar level measured result display control program according to claim 21, further making the computer execute a matching step of matching the information managed by the computer with at least a part of information managed by an external apparatus.

23. The blood sugar level measured result display control program according to claim 21 or 22, wherein the display control step includes displaying graphs of transitions of the blood sugar levels of the user on the display on the basis of the measurement data recorded on the recording unit.

24. The blood sugar level measured result display control program according to claim 23, wherein the display control step includes displaying predetermined marks corresponding to the respective condition flags associated with plural pieces of blood sugar level data indicated by the graphs in the way of being adjacent to the graphs.

25. The blood sugar level measured result display control program according to claim 21, wherein the display control step includes displaying an arithmetic value based on the measurement data recorded on the recording unit on the display.

26. The blood sugar level measured result display control program according to claim 21, wherein the display control step includes extracting a measurement data group exhibiting the same condition flag set by the condition flag setting unit from within the measurement data recorded on the recording unit, and displaying the arithmetic value about the extracted measurement data group on the display.

27. The blood sugar level measured result display control program according to claim 25 or 26, wherein the arithmetic value is an average value of the blood sugar levels of the user for a predetermined period.

28. A computer readable recording medium recorded with the blood sugar level measured result display control program according to any one of claims 21 through 27.
